(19) Europäisches Patentamt
European Patent Office
Office européen des brevets

(11) Publication number: **0 089 154 A2**

(12) # EUROPEAN PATENT APPLICATION

(21) Application number: 83301161.2

(22) Date of filing: 04.03.83

(51) Int. Cl.$^3$: C 07 C 149/42
C 07 C 147/12, C 07 C 147/14
A 61 K 31/22, A 61 K 31/135

(30) Priority: 12.03.82 GB 8207325

(43) Date of publication of application: 21.09.83 Bulletin 83/38

(84) Designated Contracting States: BE CH DE FR GB IT LI NL SE

(71) Applicant: BEECHAM GROUP PLC
Beecham House Great West Road
Brentford Middlesex(GB)

(72) Inventor: Duckworth, David Malcolm
44 Meadow Walk Walton on the Hill
Tadworth Surrey(GB)

(72) Inventor: Hindley, Richard Mark
19 Cockshot Road
Reigate Surrey(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Ethanolamine derivatives, their preparation and use in pharmaceutical compositions.**

(57) A compound of formula (I):

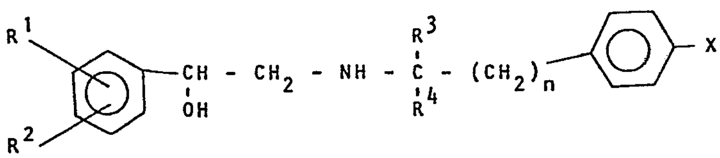

(I)

wherein;

$R^1$ is hydrogen, halogen or trifluoromethyl,
$R^2$ is hydrogen or halogen,
$R^3$ is hydrogen or methyl,
$R^4$ is hydrogen or methyl,
n is 1 or 2

X is $-Y-SR^5$, $-Y-\underset{\underset{O}{\|}}{S}-R^5$ or $-Y-\overset{\overset{O}{\|}}{\underset{\underset{O}{\|}}{S}}-R^5$

$R^5$ is optionally substituted benzyl or $C_{1-12}$ straight or branched alkyl optionally substituted by carboxy, $C_{1-6}$ alkoxycarbonyl, benzyloxycarbonyl, aminocarbonyl, mono- or di-($C_{1-6}$)alkylamino-carbonyl, hydroxy, $C_{1-6}$ alkoxy, benzyloxy, phenoxy, amino, mono- or di-($C_{1-6}$)alkylamino, $C_{1-6}$ alkylthio, benzylthio or phenylthio

provided that, when $R^5$ is alkyl substituted by hydroxy, alkoxy, alkylthio, phenylthio, benzylthio, amino, alkylamino or benzyloxy, such substituents are separated from the sulphur atom by at least two carbon atoms, and

Y is methylene or a bond;

is useful in treating obesity and hyperglycaemia.

EP 0 089 154 A2

# Ethanolamine derivatives, their preparation and use in pharmaceutical compositions

The present invention relates to derivatives of ethanolamine which have anti-obesity and/or anti-hyperglycaemic and/or anti-inflammatory activity, to processes for their production and to their use in pharmaceutical compositions.

European Published Application No. 6735 discloses compounds of formula:

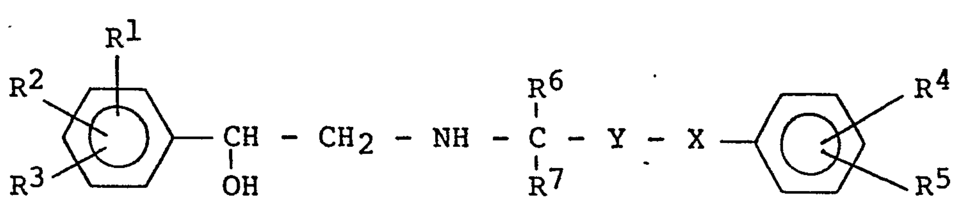

wherein:

$R^1$ is hydrogen, fluorine, chlorine, hydroxyl, hydroxymethyl, methyl, methoxy, amino, formamido, acetamido, methylsulphonamido, nitro, benzyloxy, methylsulphonylmethyl, ureido, trifluoromethyl or p-methoxybenzylamino;

$R^2$ is hydrogen, fluorine, chlorine or hydroxyl;

$R^3$ is hydrogen, fluorine, chlorine or hydroxyl;

$R^4$ is a carboxylic acid group or a salt, ester or amide thereof;

$R^5$ is hydrogen, fluorine, chlorine, methyl, methoxy, hydroxyl, or a carboxylic acid group or a salt, ester or amide thereof;

$R^6$ is hydrogen, methyl, ethyl or propyl;

$R^7$ is hydrogen, methyl, ethyl or propyl;

X is oxygen or a bond; and

Y is $C_{1-6}$ alkylene or a bond,

which possess anti-obesity and/or hypoglycaemic activity.

It has now been discovered that a class of novel ethanolamine derivatives have anti-obesity and/or anti-hyperglycaemic and/or anti-inflammatory activity. This activity is coupled with low cardiac stimulant activity for particular members of the class.

Accordingly, the present invention provides a compound of formula (I), or a salt thereof:

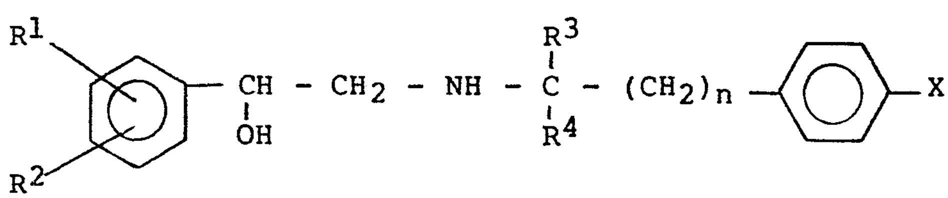

(I)

wherein;

$R^1$ is hydrogen, halogen or trifluoromethyl,

$R^2$ is hydrogen or halogen,

$R^3$ is hydrogen or methyl,

$R^4$ is hydrogen or methyl,

n is 1 or 2

X is $-Y-SR^5$, $-Y-\underset{O}{\underset{\|}{S}}-R^5$ or $-Y-\overset{O}{\overset{\|}{\underset{O}{\underset{\|}{S}}}}-R^5$

$R^5$ is optionally substituted benzyl or $C_{1-12}$ straight or branched alkyl optionally substituted by carboxy, $C_{1-6}$ alkoxycarbonyl, benzyloxycarbonyl, aminocarbonyl, mono- or di-$(C_{1-6})$alkylamino-carbonyl, hydroxy, $C_{1-6}$ alkoxy, benzyloxy, phenoxy, amino, mono- or di-$(C_{1-6})$alkylamino, $C_{1-6}$ alkylthio, benzylthio, or phenylthio

provided that, when $R^5$ is alkyl substituted by hydroxy, alkoxy, alkylthio, phenylthio, benzylthio, amino, alkylamino or benzyloxy, such substituents are separated from the sulphur atom by at least two carbon atoms, and

Y is methylene or a bond.

Preferably, $R^1$ is m-Cl or m-$CF_3$ and $R^2$ is hydrogen.

The preferred value of n is 1, and $R^3$ and $R^4$ are preferably simultaneously different.

When $R^5$ is alkyl, it is preferably $C_{1-6}$ straight or branched chain alkyl.

Salts of compounds of formula (I) are preferably pharmaceutically acceptable and include acid addition

salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicyclic acid; and, when $R^5$ includes a carboxyl group, salts of that group such as alkali metal salts, including sodium and potassium salts, alkaline earth metal salts, including calcium and magnesium salts, and ammonium salts.

Salts of compounds of formula (I) need not be pharmaceutically acceptable as they are also useful in the preparation of other compounds of formula (I) and in the separation of stereoisomers of compounds of formula (I) when the salt ion is also optically active.

Compounds of formula (I) have at least one asymmetric carbon atom, ie the carbon atom bearing the hydroxyl and substituted phenyl groups, and, when $R^3$ and $R^4$ are different, the carbon atom bearing $R^3$ and $R^4$ is also asymmetric. The compounds may, therefore, exist in at least two and often four stereoisomeric forms. The present invention encompasses all stereoisomers of the compounds of formula (I) whether free from other stereoisomers or admixed with other stereoisomers in any proportion and thus includes, for instance, racemic mixtures of enantiomers.

Preferably, the carbon atom bearing the hydroxyl and substituted phenyl groups has the <u>R</u>-configuration.

The most potent compounds of formula (I) are those wherein $R^3$ and $R^4$ are different and both asymmetric carbon atoms are in the <u>R</u>-configuration.

The absolute configuration of any compound of formula (I) may be determined by conventional X-ray crystallographic techniques.

The present invention provides a process for producing a compound of formula (I), or a salt thereof, which process comprises reducing an oxo-group and/or double bond and/or cleaving an N-benzyl group of a compound of formula (II):

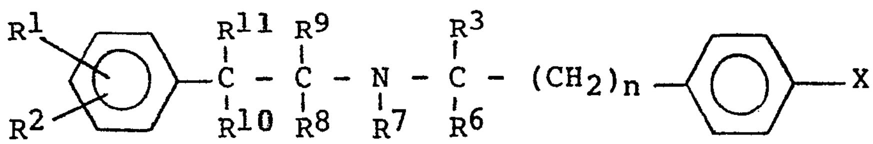

(II)

wherein $R^1$, $R^2$, $R^3$, X and n are as defined in relation to formula (I),

$R^6$ is a group $R^4$ as defined in relation to formula (I) or together with $R^7$ forms a bond,

$R^7$ is hydrogen or benzyl or together with $R^6$ or $R^8$ forms a bond,

$R^8$ is hydrogen or together with $R^9$ forms an oxo-group or together with $R^7$ forms a bond,

$R^9$ is hydrogen or together with $R^8$ forms an oxo-group,

$R^{10}$ is hydroxyl or together with $R^{11}$ forms an oxo-group,

$R^{11}$ is hydrogen or together with $R^{10}$ forms an oxo-group

provided that there is no more than one oxo-group represented by any of $R^8$ to $R^{11}$;and optionally thereafter forming a salt of the compound of formula (I) so formed and/or converting the compound of formula (I) so formed into a further compound of formula (I).

Conversion of a compound of formula (I) into a further compound of formula (I) may be effected by, for instance, reducing an ester to the corresponding alcohol or forming an amide from an ester, using conventional methods.

Where there are two or more reducible moieties in the compound of formula (II) these may be reduced separately in any order or simultaneously.

The aforementioned reductions may be effected by conventional chemical methods or by catalytic methods. Suitably, chemical reduction may be effected with lithium aluminium hydride, sodium cyanoborohydride or sodium borohydride. Catalytic hydrogenation may be carried out using catalysts such as palladium on charcoal, or platinum, for instance, as platinum oxide.

Preferably reduction of thioethers of formula (II) is carried out using conventional chemical reagents.

Reduction by sodium borohydride is conveniently effected in a lower alkanolic solvent such as methanol or ethanol. The reaction is generally carried out at from 0-20°C.

Reduction by lithium aluminium hydride is conveniently effected in a dry, ether solvent such as diethyl ether or tetrahydrofuran at ambient or elevated temperatures.

Catalytic reduction is conveniently effected in a conventional hydrogenation solvent such as a lower alkanol, for instance ethanol. The hydrogenation is generally carried out under hydrogen gas at about 1 atmosphere pressure and at ambient or elevated temperatures.

Reduction of a compound of formula (II) wherein $R^7$ is benzyl is conveniently effected by catalytic hydrogenation, preferably using palladium on charcoal as catalyst when X is a sulphoxide or sulphone group.

Preferred aspects of the process comprise reducing a compound of formula (IIA):

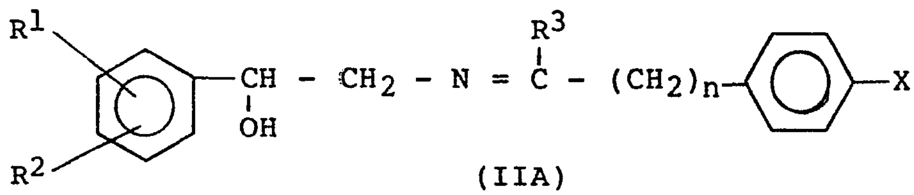

(IIA)

or reducing a compound of formula (IIB):

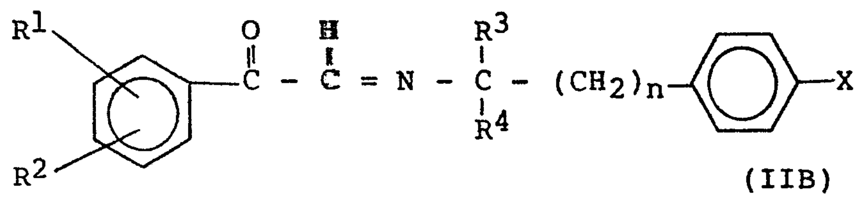

(IIB)

or reducing a compound of formula (IIC):

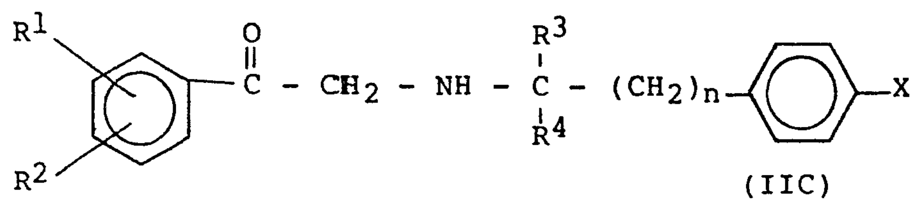

(IIC)

or reducing a compound of formula (IID)

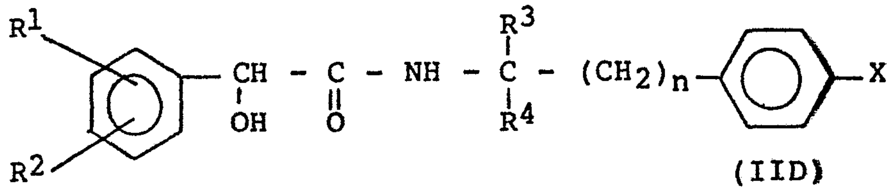

(IID)

or reducing a compound of formula (IIE):

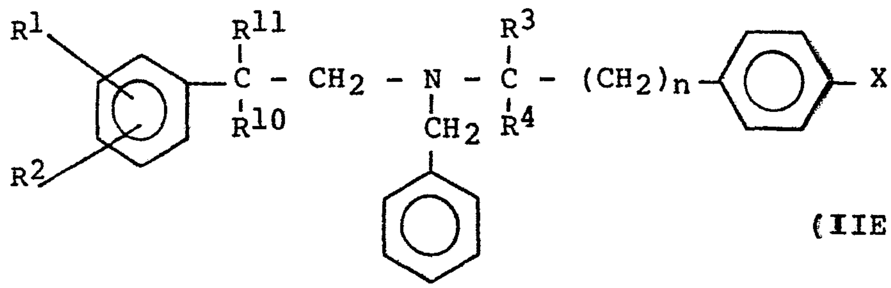

(IIE)

wherein $R^1$, $R^2$, $R^3$, $R^4$, X and n are as defined in relation to formula (I) and $R^{10}$ and $R^{11}$ are as defined in relation to formula (II).

The present invention also provides another process for producing a compound of formula (I) or a salt thereof, which process comprises reacting a compound of formula (III):

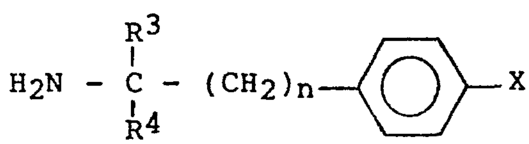

(III)

wherein $R^3$, $R^4$, X and n are as defined in relation to formula (I);

with a compound of formula (IV):

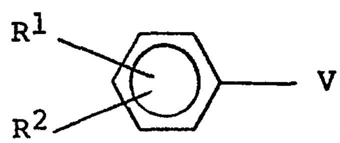

(IV)

wherein $R^1$ and $R^2$ are as defined in relation to formula (I), and V is a group capable of reacting with the amine of formula (III) thus forming a compound of formula (I), and optionally thereafter forming a salt of the compound of formula (I) so formed.

Typical examples of compounds of formula (IV) are compounds of formulae (IVA) and (IVB):

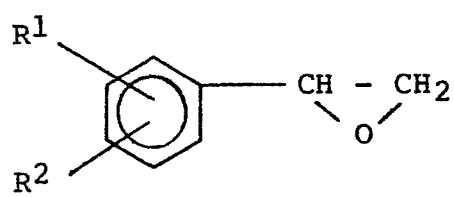

(IVA)

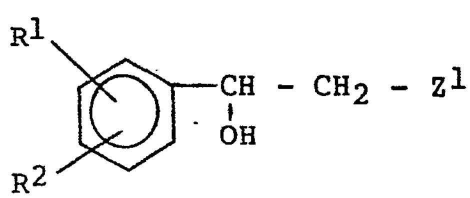

(IVB)

wherein $R^1$ and $R^2$ are as defined in relation to formula (I) and $Z^1$ is a leaving group, preferably halogen or tosyloxy.

The reaction of a compound of formula (III) with a compound of formula (IVA) is conveniently effected in a solvent such as a lower alkanol, preferably ethanol.

The reaction of a compound of formula (III) with a compound of formula (IVB) is conveniently conducted in a solvent, such as dimethyl sulphoxide, at elevated temperature, preferably about 50°C, for about 3 days.

The present invention provides a further process for the production of compounds of formula (I) or salts thereof, which process comprises reacting a compound of formula (V):

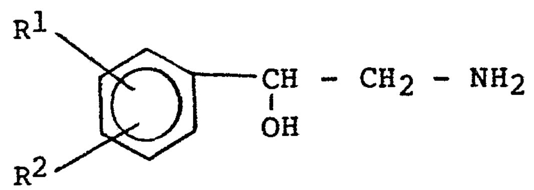

(V)

wherein $R^1$ and $R^2$ are as defined in relation to formula (I);

with a compound of formula (VI):

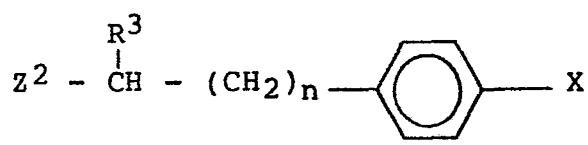

(VI)

wherein $R^3$, n and X are as defined in relation to formula (I) and $Z^2$ is a leaving group preferably halogen or tosyloxy.

The preparation of compounds of formula (V) is described in Published European Patent Application No. 0 021 636.

The reaction of a compound of formula (V) with a compound of formula (VI) is conveniently effected in a solvent such as dimethyl sulphoxide at an elevated temperature, preferably about 50°C for about two or three days.

A preferred process for producing compounds of formula (I) comprises the reduction of a compound of formula (IIA), especially using sodium borohydride in methanol at ambient temperatures.

The salts of compounds of formula (I) may be produced by treating the compound of formula (I) with the appropriate acid, or when the compound is a carboxylic acid by treatment with an appropriate base.

Compounds of formula (I) and salts thereof, produced by the above processes, may be recovered by conventional methods.

Compounds of formula (II) may be produced by reacting a compound of formula (III) as hereinbefore defined;

with a compound of formula (VII):

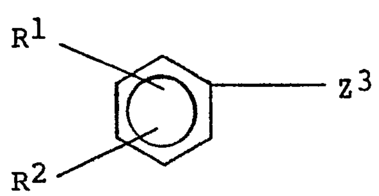

(VII)

wherein $R^1$ and $R^2$ are as defined in relation to formula (I) and $Z^3$ is a group which is capable of reacting with the amine of formula (III) thus forming a compound of formula (II). Typical examples of compounds of formula (VII) are:

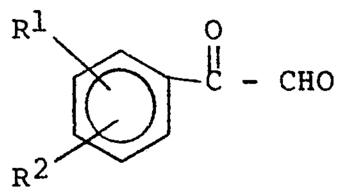

(VIIA)

or its hydrate or hemi-acetal of a lower alkanol;

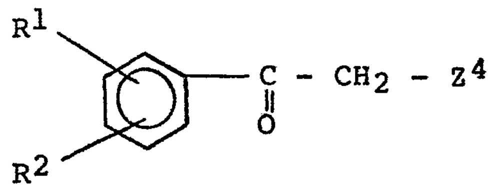

(VIIB)

wherein $Z^4$ is a leaving group, preferably bromine;

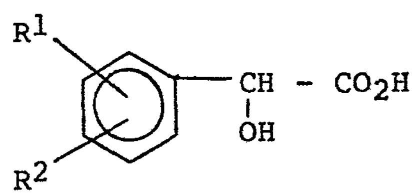

(VIIC)

wherein $R^1$ and $R^2$ are as defined in relation to formula (I).

Conventional conditions suitable for use with the particular compound of formula (VII) may be used for this reaction. Thus the reaction of a compound of formula (VIIA) with a compound of formula (III) is conveniently conducted at elevated temperatures under conditions resulting in the removal of the water formed during the reaction. A particularly suitable method is to perform the reaction in a solvent, such as benzene, under reflux and to remove the water azeotropically using a Dean and Stark trap.

The reaction of a compound of formula (VIIB) with a compound of formula (III) is conveniently conducted in a polar organic solvent such as acetonitrile or butanone, at an elevated temperature, for instance under reflux.

The reaction of a compound of formula (VIIC) with a compound of formula (III) is conveniently conducted under standard peptide formation reaction conditions.

Alternatively a compound of formula (II) may be prepared by reacting a compound of formula (VIII):

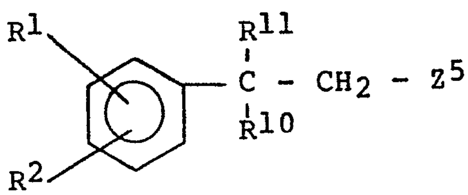

(VIII)

wherein $R^1$, $R^2$, $R^{10}$ and $R^{11}$ are as defined in relation to formula (II);

and $Z^5$ is a leaving group, preferably halogen or tosyloxy;

with a compound of formula (IX):

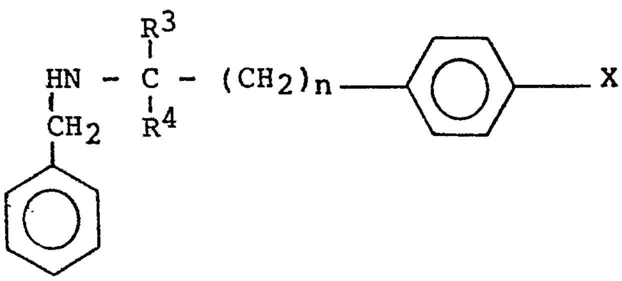

wherein $R^3$, $R^4$, n, and X are as defined in relation to formula (I).

A particularly preferred process for producing compounds of formula (IID) is by reacting a compound of formula (V) as hereinbefore defined with a compound of the formula (X):

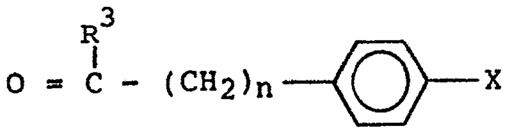

(X)

wherein $R^3$, X and n are as defined in relation to formula (I).

The reaction of a compound of formula (V) with a compound of formula (X) is conveniently effected under conditions which result in the removal of water formed during the reaction. A particularly suitable method is to perform the reaction in a solvent, such as benzene, under reflux and to remove the water azeotropically using a Dean and Stark trap.

It is often convenient to prepare the compound of formula (II) and reduce it, in situ, to the desired compound of formula (I) without isolation of the compound of formula (II).

Compounds of formula (I) having a single asymmetric carbon atom may, if desired, be separated into individual enantiomers by conventional means, for example, by the use of an optically active acid as a resolving agent. Those compounds of formula (I) having two asymmetric carbon atoms may be separated into diastereoisomeric pairs of enantiomers by, for example, fractional crystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in "Topics in Stereochemistry", Vol. 6, Wiley Interscience, 1971, Allinger, N.L., and Eliel, W.L. Eds.

Alternatively any enantiomer of a compound of formula (I) may be obtained by stereospecific synthesis using optically pure starting materials of known configuration.

By using single enantiomers of a compound of formula (III) and of a compound of formula (VIIC) a stereospecific synthesis of a compound of formula (II) is achieved. This may then be reduced to a compound of formula (I) without altering the configuration of the two asymmetric carbon atoms. Thus, for example, reaction of a compound of formula (III) with the R-absolute configuration and a compound of formula (VIIC) with the R-absolute configuration would afford a compound of formula (II) and, on reduction, a compound of formula (I) with the RR-absolute configuration.

By reacting a single enantiomer of a compound of formula (III) with a single enantiomer of a compound of formula (IVA) or (IVB), the direct stereospecific synthesis of a single enantiomer of a compound of formula (I) is effected. Thus, for example, reaction of a compound of formula (III) with the R-absolute configuration with a compound of formula (IVA) with the R-absolute configuration would afford a compound of formula (I) with the RR-absolute configuration.

A compound of formula (I) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt

thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or the like.

Typical carriers may, therefore, comprise such agents as microcrystalline cellulose, starch, sodium starch glycollate, polyvinylpyrrolidone, polyvinyl-polypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating obesity or hyperglycaemia in humans or mammals, which method comprises administering an

effective, non-toxic amount of a compound of formula (I) or a pharmaceutically acceptable salt thereof to obese or hyperglycaemic humans or mammals.

In treating hyperglycaemic or obese humans the drug may be taken in doses, such as those described above, one to six times a day in a manner such that the total daily dose for a 70 kg adult will generally be about 0.1 to 3000 mg, and more usually about 1 to 1500 mg.

In treating hyperglycaemic or obese animals, especially dogs, the drug may be administered by mouth, usually once or twice a day and at about 0.025 mg/kg to 10 mg/kg, for example 0.1 mg/kg to 2 mg/kg.

The invention also provides a method for treating inflammation in humans, which comprises topically administering an effective, non-toxic amount of a compound of formula (I) or a pharmaceutically acceptable salt thereof, to a human in need of such treatment.

Conveniently, the drug may be administered as a pharmaceutical composition as hereinbefore defined, and this forms a particular aspect of the present invention.

The invention will now be illustrated with reference to the following Examples and the preparation of intermediates will be illustrated with reference to the Descriptions.

As used in the Examples, the term "diastereoisomer" refers to a racemic pair of enantiomers.

Example 1

N-[2-(4-(2-Carbomethoxyethylthio)phenyl)-1-methylethyl] 2-hydroxy-2-(3-trifluoromethylphenyl)ethanamine

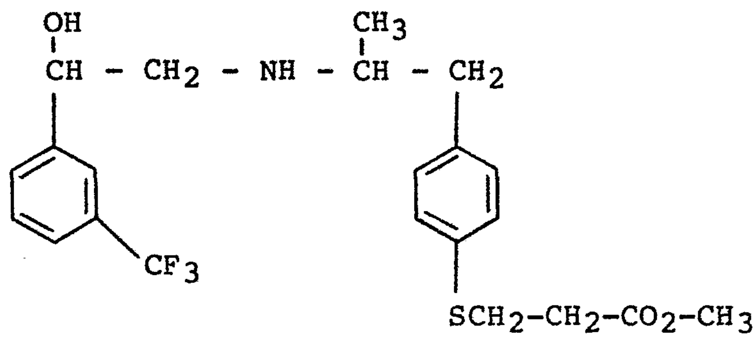

A mixture of 1-[4-(2-carbomethoxyethylthio)phenyl] propan-2-one (3.05 g) and 2-hydroxy-2-(3-trifluoromethylphenyl)ethanamine (2.48 g) in dry benzene was heated under reflux using a Dean and Stark head for 2 hours, cooled and the solvent evaporated. The residue was dissolved in methanol (100 ml), cooled to 0°C, and treated portionwise with sodium borohydride (1.5 g) with stirring. The mixture was stirred for a further hour at 0°C, and, the solvent was removed under reduced pressure. Water was added to the residue which was then extracted with ethyl acetate. The combined organic extracts were dried and concentrated by evaporation. Chromatography of the residue on silica gel (eluted with 5% methanol in dichloromethane) gave N-[2-(4-(2-carbomethoxyethylthio)phenyl)-1-methylethyl] 2-hydroxy-2-(3-trifluoromethylphenyl)ethanamine, mp 78-82°C (ethyl acetate-hexane) as a 80:20 mixture of diastereoisomers.

$^1$H nmr; $\tau$ ($CDCl_3$) 8.95 (3H, d, J=6Hz), 6.7-7.8 (11H, complex; 2H exchange on addition of $D_2O$), 6.35 (3H, s), 5.4 (1H, dd), 2.2-3.0 (8H, complex).

0089154

Example 2

N-[2-(4-Methylthiophenyl)-1-methylethyl]-2-hydroxy-2-(3-chlorophenyl)ethanamine

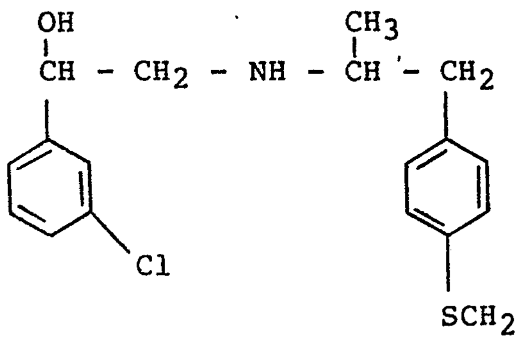

A mixture of 1-(4-methylthiophenyl)propan-2-one (3.0 g) and 2-hydroxy-2-(3-chlorophenyl)ethanamine (3.0 g) in dry benzene was heated under reflux using a Dean and Stark apparatus for 2 hours. The reaction mixture was cooled, and the solvent evaporated. The residue was dissolved in methanol (100 ml) and sodium borohydride (5.0 g) was added portionwise at ambient temperature. The reaction mixture was stirred at ambient temperatures for a further 30 mins. Water was added, and the suspension extracted with dichloromethane. The organic extracts were dried (magnesium sulphate) and the solvent evaporated. Chromatography of the residue on silica gel (eluted with 5% methanol-dichloromethane) followed by crystallisation from ether-hexane gave N-[2-(4-methylthiophenyl)-1-methylethyl]-2-hydroxy-2-(3-chlorophenyl)ethanamine (1.1 g), mp 97-105°C as a 38:62 mixture of diastereoisomers.

$^1H$ nmr; $\tau$ ($CDCl_3$) 8.9 (3H, d, J=6Hz), 7.5 (3H, s), 6.8-7.4 (7H, complex, 2H exchange on $D_2O$ addition), 5.3 (1H, m), 2.5-3.1 (8H,m).

Example 3

N-[2-(4-Isopropylthiophenyl)-1-methylethyl]-2-hydroxy-2-(3-chlorophenyl)ethanamine hydrochloride

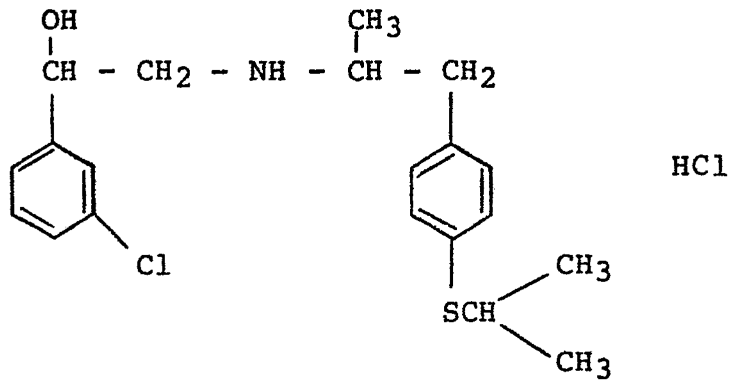

1-(4-Isopropylthiophenyl)propan-2-one (1.8 g) and 2-hydroxy-2-(3-chlorophenyl)ethanamine (1.58 g) were reacted together according to the procedure described in Example 2. Column chromatography on silica gel gave an oil which was converted to its hydrochloride salt and crystallised from methanol/ethylacetate/diethyl ether to give N-[2-(4-Isopropylthiophenyl)-1-methylethyl]-2-hydroxy-2-(3-chlorophenyl)ethanamine hydrochloride, mp 78-86°C, as a 52:48 mixture of diastereoisomers.

$^1H$ nmr; $\tau$ ($d_6$-DMSO) 8.8 (9H, m), 6.3-7.4 (6H, m), 4.9 (1H, m), 3.65 (1H, exchangeable), 2.4-2.9 (8H, m), 0.6 (2H, exchangeable).

Example 4

N-[2-(4-Benzylthiophenyl)-1-methylethyl]-2-hydroxy-2-(3-chlorophenyl)ethanamine

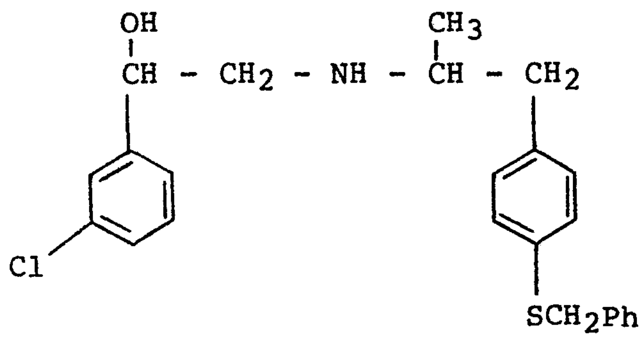

N-[2-(4-Benzylthiophenyl)-1-methylethyl]-2-hydroxy-2-(3-chlorophenyl)ethanamine was prepared from 1-(4-benzylthiophenyl)propan-2-one (3.54 g) and 2-hydroxy-2-(3-chlorophenyl)ethanamine (2.37 g) by an analogous procedure to that described in Example 2. The product, an oil, obtained by silica gel chromatography was triturated with ether-petrol, to give N-[2-(4-benzylthiophenyl)-1-methylethyl]-2-hydroxy-2-(3-chlorophenyl)ethanamine, mp 110-115°C as a 45:55 mixture of diastereoisomers.

$^1H$ nmr; $\tau$ ($CDCl_3$ + $d_6$-DMSO) 9.0 (3H, d), 7.0-7.6 (7H, m; 2H exchange with $D_2O$), 5.95 (2H, s), 5.4 (1H, m), 2.5-3.25 (13H, m).

Example 5

N-[2-(4-t-Butylthiophenyl)-1-methylethyl]-2-hydroxy-2-(3-chlorophenyl)ethanamine hydrobromide

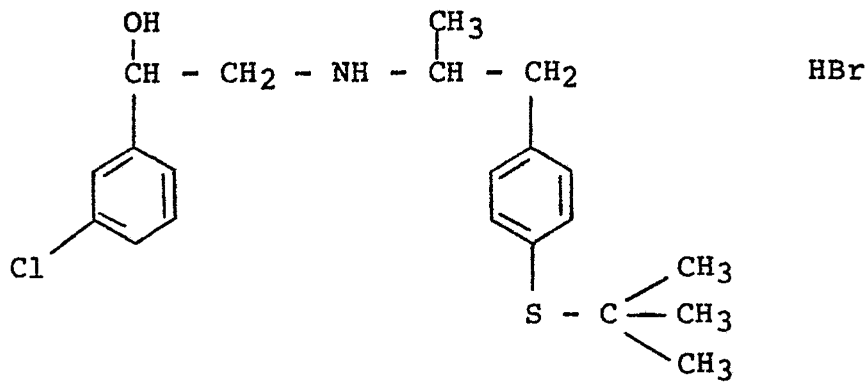

N-[2-(4-t-Butylthiophenyl)-1-methylethyl]-2-hydroxy-2-(3-chlorophenyl)ethanamine was prepared from 1-(4-t-butylthiophenyl)-propan-2-one (2.38 g) and 2-hydroxy-2-(3-chlorophenyl)ethanamine (1.71 g) by an analogous procedure to that described in Example 2. Conversion of the oil obtained from column chromatography to its hydrobromide salt in ether gave N-[2-(4-t-butylthiophenyl)-1-methylethyl]-2-hydroxy-2-(3-chlorophenyl)ethanamine hydrobromide as a white solid, mp 172-82°C, as a 86:14 mixture of diastereoisomers.

$^1H$ nmr; $\tau$ ($d_6$-DMSO) 8.65-9.0 (12H, s on d), 6.2-7.35 (6H, complex; 1H exchangeable with $D_2O$), 4.9 (1H, m), 2.35-2.8 (8H, m), 0.8-1.5 (2H, exchangeable with $D_2O$).

Example 6

N-[2-(4-n-Heptylthiophenyl)-1-methylethyl]-2-hydroxy-2-(3-chlorophenyl)ethanamine hydrobromide

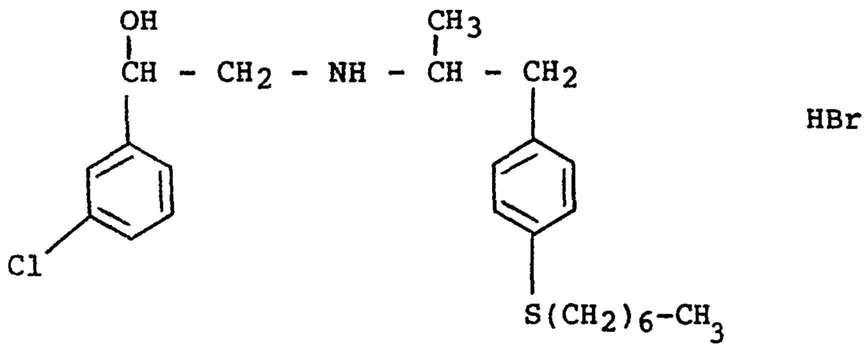

N-[2-(4-n-Heptylthiophenyl)-1-methylethyl]-2-hydroxy-2-(3-chlorophenyl)ethanamine was prepared from 1-(4-n-heptylthiophenyl)-propan-2-one (2.8 g) and 2-hydroxy-2-(3-chlorophenyl)ethanamine (1.71 g) by an analogous procedure to that described in Example 2. The oil obtained from column chromatography was converted to its hydrobromide salt to give N-[2-(4-n-heptylthiophenyl)-1-methylethyl]-2-hydroxy-2-(3-chlorophenyl)ethanamine hydrobromide, mp 125-134°C, as a 91:9 mixture of diastereoisomers.

$^1H$ nmr; $\tau$ ($d_6$-DMSO) 8.2-9.3 (16H, complex), 6.4-7.35 (7H, complex), 4.9 (1H, m), 3.7 (1H, exchangeable with $D_2O$), 2.3-2.85 (8H, complex), 0.9-1.4 (2H, exchangeable with $D_2O$).

Example 7

N-[2-(4-Methylsulphonylphenyl)-1-methylethyl]-2-hydroxy-2-(3-trifluoromethylphenyl)ethanamine

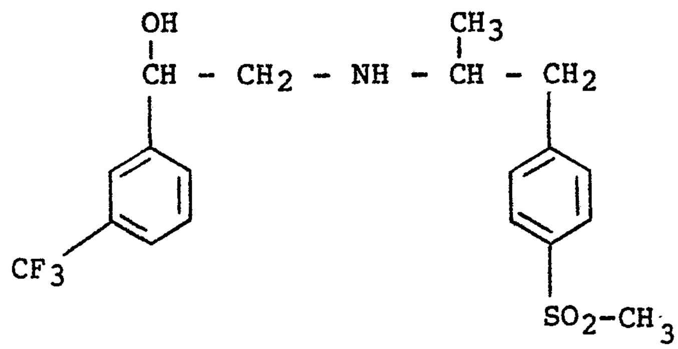

N-[2-(4-Methylsulphonylphenyl)-1-methylethyl]-2-hydroxy-2-(3-trifluoromethylphenyl)ethanamine, mp 101-3°C (hexane) was prepared from 1-(4-methylsulphonylphenyl)propan-2-one (1.7 g) and 2-hydroxy-2-(3-trifluoromethylphenyl)ethanamine (1.64 g) by an analogous procedure to that described in Example 1 and was obtained as a 48:52 mixture of diastereoisomers.

$^1H$ nmr; ($d_6$-DMSO) 9.05 (3H, d), 8.35 (1H, exchangeable with $D_2O$), 6.9-7.4 (5H, m), 6.8 (3H, s), 5.3 (1H, m), 4.55 (1H, exchangeable with $D_2O$), 2.55 (2H, d), 2.2-2.5 (4H, m), 2.15 (2H, d).

Example 8

N-[2-(4-Isopropylsulphonylphenyl)-1-methylethyl]-2-hydroxy-2-(3-trifluoromethylphenyl)ethanamine

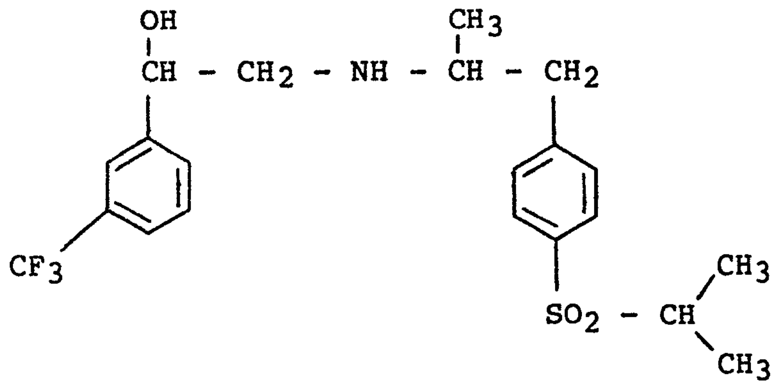

N-[2-(4-Isopropylsulphonylphenyl)-1-methylethyl]-2-hydroxy-2-(3-trifluoromethylphenyl)ethanamine, mp 88-94°C (hexane) was prepared from 1-(4-isopropylsulphonylphenyl)propan-2-one (2.0 g) and 2-hydroxy-2-(3-trifluoromethylphenyl)ethanamine (1.7 g) by an analogous procedure to that described in Example 1 and was obtained as a 46:54 mixture of diastereoisomers.

$^1$H nmr; ($CDCl_3$) 8.95 (3H, d), 8.7 (6H, d), 7.7 (2H, broad s, exchangeable with $D_2O$), 6.6-7.5 (6H, m), 5.35 (1H, m), 2.65 (2H, d), 2.3-2.6 (4H, m), 2.2 (2H, d).

Example 9

N-[2-(4-{3-Hydroxypropylsulphonyl} phenyl)-1-methylethyl] -2-hydroxy-2-(3-trifluoromethylphenyl)-ethanamine

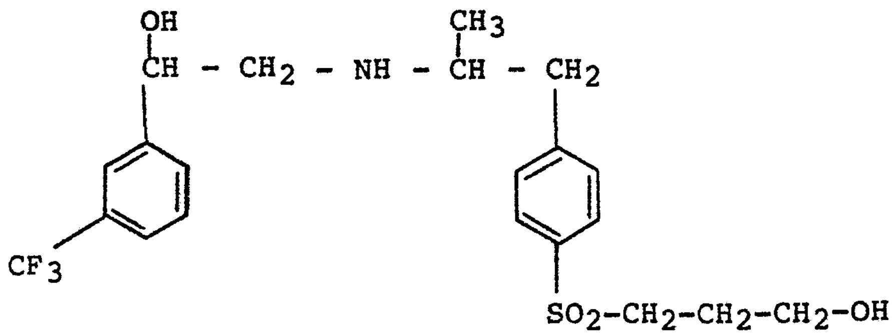

N-[2-(4-{3-Hydroxypropylsulphonyl} phenyl)-1-methylethyl]-2-hydroxy-2-(3-trifluoromethylphenyl) ethanamine, mp 84-9°C (ethyl acetate-diethyl ether), was obtained from 1-[4-(2-carbomethoxyethylsulphonyl)-phenyl]propan-2-one (1.6 g) and 2-hydroxy-2-(3-trifluoromethylphenyl)ethanamine (1.2 g) by an analogous procedure to that described in Example 1 (a large excess of sodium borohydride (2.5 g) was employed; the reaction was conducted at ambient temperature).

$^1H$ nmr; $\tau$ ($CDCl_3$) 8.8 (3H, d), 8.1 (2H, m), 6.6-7.7 (10H, complex; 3H exchange with $D_2O$), 6.4 (2H, m), 5.4 (1H, m), 2.3-2.75 (6H· complex), 2.15 (2H, d).

Example 10

N-[2-(4-{2-Carbomethoxyethylsulphonyl}phenyl)-1-methylethyl] -2-hydroxy-2-(3-chlorophenyl)ethanamine

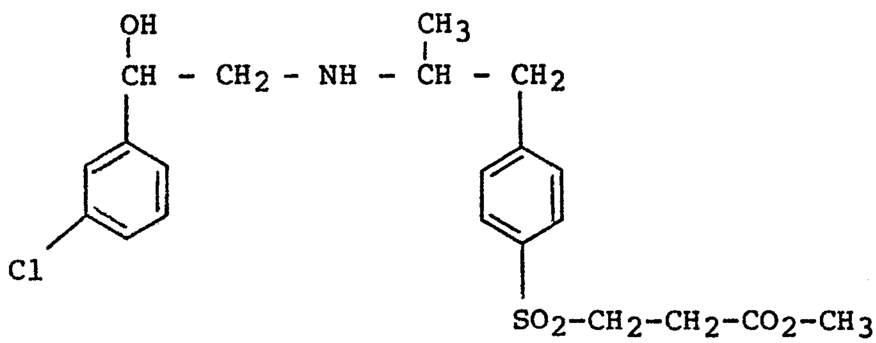

A mixture of 1-[4-(2-carbomethoxyethylsulphonyl)-phenyl]propan-2-one (4.2 g) and 2-hydroxy-2-(3-chlorophenyl)ethanamine (2.7 g) in benzene was heated under reflux using a Dean and Stark head for one hour, then cooled and the solvent evaporated. The residue was dissolved in methanol (100 ml) and hydrogenated over platinum at atmospheric pressure. After hydrogen uptake had ceased, the solution was filtered and evaporated to dryness. Chromatography of the residue on silica gel (5% methanol in dichloromethane as eluent) gave N-[2-(4-{2-Carbomethoxyethylsulphonyl} phenyl)-1-methylethyl]-2-hydroxy-2-(3-chlorophenyl)-ethanamine, mp 75-90°C (ethyl acetate-diethyl ether).

$^1H$ nmr; $\tau$ ($CDCl_3$) 8.95 (3H, d), 6.9-7.7 (9H complex; 2H exchange with $D_2O$), 6.65 (2H, t), 6.4 (3H, s), 5.45 (1H, m), 2.5-2.95 (6H, complex), 2.2 (2H, d).

Example 11

N-[2-(4-Methylsulphonylmethylphenyl)-1-methylethyl]-2-hydroxy-2-(3-trifluoromethylphenyl)ethanamine

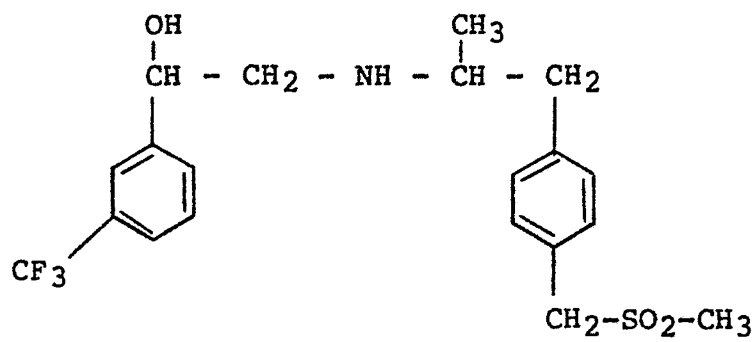

N-[2-(4-Methylsulphonylmethylphenyl)-1-methylethyl]-2-hydroxy-2-(3-trifluoromethylphenyl)ethanamine, mp 130-42°C (methanol) was obtained as a 57:43 mixture of diastereoisomers from 1-(4-methylsulphonylmethylphenyl)propan-2-one (5.2 g) and 2-hydroxy-2-(3-trifluoromethylphenyl)ethanamine (4.2 g) by an analogous procedure to that described in Example 1.

$^1$H nmr; $\tau$ ($CDCl_3$) 8.95 (3H, d), 7.0-7.7 (10H, complex; 2H exchange with $D_2O$), 5.8 (2H, s), 5.4 (1H, m), 2.3-2.95(8H, complex).

Example 12

N-[2-(4-Isopropylsulphonylmethylphenyl)-1-methylethyl] -2-hydroxy-2-(3-trifluoromethylphenyl)ethanamine

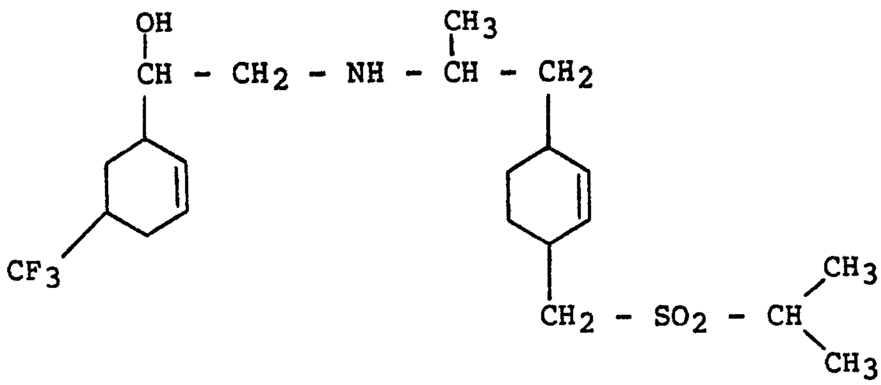

N-[2-(4-Isopropylsulphonylmethylphenyl)-1-methylethyl]-2-hydroxy-2-(3-trifluoromethylphenyl) ethanamine, mp 129-40°C, was obtained as a 52:48 mixture of diastereoisomers from 1-(4-isopropylsulphonylmethylphenyl)propan-2-one (1.0 g) and 2-hydroxy-2-(3-trifluoromethylphenyl)ethanamine (0.8 g) by an analogous procedure to that described in Example 1.

$^{1}H$ nmr; $\tau$ ($d_6$-DMSO) 9.05 (3H, d), 8.7 (6H, d), 6.65-7.5 (8H, complex; 2H exchange with $D_2O$), 5.6 (2H, s), 5.3 (1H, t), 2.6-2.95 (4H, dd), 2.15-2.5 (4H, complex).

0089154

EXAMPLE 13

N-[2-(4-Isopropylthiomethylphenyl)-1-methyethyl]-2-hydroxy-2-(3-chlorophenyl)ethanamine

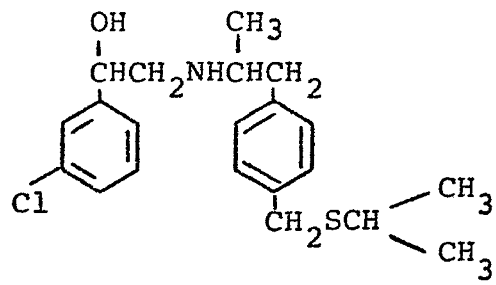

N-[2-(4-Isopropylthiomethylphenyl)-1-methylethyl]-2-hydroxy-2-(3-chlorophenyl)ethanamine, m.p. 85-97$^{o}$ C (hexane) was obtained as a 25:75 mixture of diastereoisomers from 1-(4-isopropylthiomethylphenyl)propan-2-one (0.47g) and 2-hydroxy-2-(3-chlorophenyl)ethanamine (0.36g) by an analogous procedure to that described in Example 2.

$^{1}H$ nmr; $\tau(CDCl_3 + D_2O)$:

8.9 (3H,d), 8.72 (6H,d), 6.9-7.5 (6H,m), 6.25 (2H,s), 5.45 (1H,m), 2.5-2.95 (8H,m).

Synthesis of a sample enriched in the other diastereoisomer is described in Example 14.

EXAMPLE 14

N-[2-(4-Isopropylthiomethylphenyl)-1-methylethyl]-2-hydroxy-2-(3-chlorophenyl)ethanamine hydrobromide

N-[2-(4-Isopropylthiomethylphenyl)-1-methylethyl]-2-hydroxy-2-(3-chlorophenyl)ethanamine, obtained as an oil after column chromatography from Example 13, was converted into its hydrobromide salt in ether to give N-[2-(4-isopropylthiomethylphenyl)-1-methylethyl]-2-hydroxy-2-(3-chlorophenyl)ethanamine hydrobromide as a white solid, m.p. 168-170$^{o}$C(EtoAc - MeOH), as a 96:4 mixture of diastereoisomers.

$^{1}$H nmr; $\tau$($d_6$-DMSO):

8.9 (3H,d), 8.8 (6H,d), 6.4-7.4 (6H,m), 6.25 (2H,s), 4.95 (1H,m), 3.7 (1H, bs, replaceable by $D_2O$), 2.4-2.9 (8H,m), 0.8-1.6 (2H, broad s, replaceable by $D_2O$).

EXAMPLE 15

N-[2-(4-t-Butylsulphonylphenyl)-1-methylethyl]-2-hydroxy-2-(3-chlorophenyl)ethanamine hydrobromide

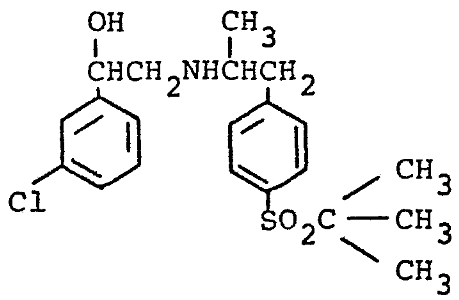

.HBr

N-[2-(4-t-Butylsulphonylphenyl)-1-methylethyl]-2-hydroxy-2-(3-chlorophenyl)ethanamine was prepared from 1-(4-t-butylsulphonylphenyl)propan-2-one (2.97g) and 2-hydroxy-2-(3-chlorophenyl)ethanamine (2.0g) by an analogous procedure to that described in Example 2. Conversion of the oil obtained from column chromatography to its hydrobromide salt in ether gave N-[2-(4-t-butylsulphonylphenyl)-1-methylethyl]-2-hydroxy-2-(3-chlorophenyl)ethanamine hydrobromide as a white solid, m.p. 170-173$^{o}$C (EtoAc, MeOH), as a 45:55 mixture of diastereoisomers.

$^{1}$H nmr; τ ($d_6$-DMSO);

8.5-9.1 (12H, s on d), 6.2-7.3 (5H,m), 4.85 (1H,m), 3.66 (1H, exchangeable with $D_2O$), 2.0-2.7 (8H,m), 0.5-1.5 (2H, broad, exchangeable with $D_2O$).

EXAMPLE 16

N-[2-(4-n-Heptylsulphonylphenyl)-1-methylethyl]-2-hydroxy-2-(3-chlorophenyl)ethanamine

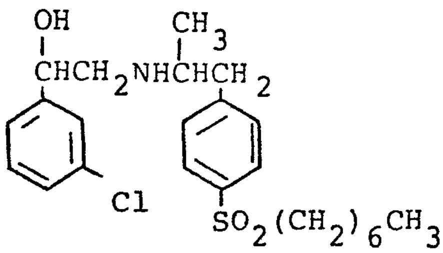

N-[2-(4-n-Heptylsulphonylphenyl)-1-methylethyl]-2-hydroxy-2-(3-chlorophenyl)ethanamine, m.p. 65-80°C (hexane) was prepared from 1-(4-n-heptylsulphonylphenyl)propan-2-one (3.56g) and 2-hydroxy-2-(3-chlorophenyl)ethanamine (2.06g) by an analogous procedure to that described in Example 2 and was obtained as a 45:55 mixture of diastereoisomers.

$^1H$ nmr; $\tau(CDCl_3)$:

9.15 (3H,t), 8.9 (3H,d), 8.0-8.8 (10H,m), 6.8-8.0 (7H,m + 2H exchangeable with $D_2O$), 5.4 (1H,m), 2.5-2.85 (6H,m), 2.1 (2H,d).

EXAMPLE 17

N-[2-(4-Isopropylthiophenyl)-1-methylethyl]-2-hydroxy-2-phenylethanamine

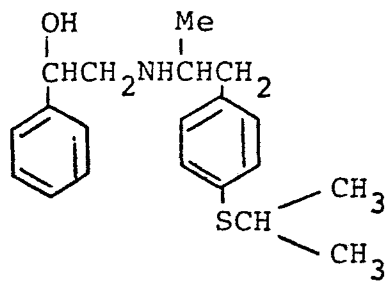

1-(4-Isopropylthiophenyl)propan-2-one (4.15g) and 2-hydroxy-2-phenylethanamine (2.74g) were reacted together according to the procedure described in Example 2. Column chromatography on silica gel gave a solid which on crystallisation from diethyl ether/hexane gave N-[2-(4-isopropylthiophenyl)-1-methylethyl]-2-hydroxy-2-phenylethanamine, m.p. 82-89$^{o}$C as a 19:81 mixture of diastereoisomers.

$^{1}$H nmr $\tau$($CDCl_3$) :

8.95 (3H,d), 8.72 (6H,d), 6.45-7.80 (8H, complex, 2H exchangeable), 5.35 (1H,q), 2.5-3.0 (9H, complex).

0089154

EXAMPLE 18

(RS:SR)-N-[2-(4-Methylthiophenyl)-1-methylethyl]-2-hydroxy-2-(3-chlorophenyl)ethanamine

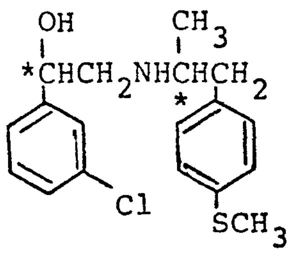

RS,SR Diastereoisomer

N-[2-(4-Methylthiophenyl)-1-methylethyl]-2-hydroxy-2-(3-chlorophenyl)ethanamine was obtained as a mixture of diastereoisomers by the procedure described in Example 2. Crystallisation from diethyl ether/hexane followed by recrystallisation from diethyl ether gave (RS,SR)-N-[2-(4-methylthiophenyl)-1-methylethyl]-2-hydroxy-2-(3-chlorophenyl)ethanamine, m.p. 112-5$^{o}$C as a 6:94 mixture of diastereoisomers.

EXAMPLE 19

(RR,SS)-N-[2-(4-Methylthiophenyl)-1-methylethyl]-2-hydroxy-1-(3-chlorophenyl) ethanamine hydrochloride

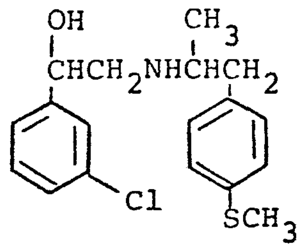

.HCl RR,SS diastereoisomer

The crystallisation mother liquors from Example 18 were combined and the solvent was removed under reduced pressure. The residue dissolved in diethyl ether was treated with a solution of hydrogen chloride in diethyl ether. The solvent was removed under reduced pressure and the residue was crystallised from diethyl ether/hexane to give (RR,SS)-N-[2-(4-methylthiophenyl)-1-methylethyl]-2-hydroxy-2-(3-chlorophenyl)ethanamine hydrochloride, m.p. 127-9°C of >95% diastereoisomeric purity.

EXAMPLE 20

N-[2-(4-Methylsulphinylphenyl)-1-methylethyl]-2-hydroxy-2-(3-chlorophenyl) ethanamine

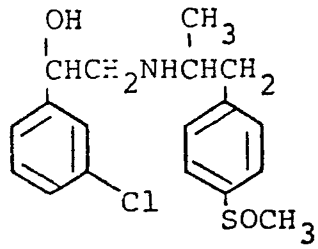

1-(4-Methylsulphinylphenyl)propan-2-one (3.2g) and 2-hydroxy-2-(3-chlorophenyl)ethanamine (3.0g) were reacted together according to the procedure described in Example 2. Column chromatography on silica gel and crystallisation from ethyl acetate/diethyl ether/hexane gave N-[2-(4-methylsulphinylphenyl)-1-methylethyl]-2-hydroxy-2-(3-chlorophenyl) ethanamine, m.p. 101-108$^{o}$C, as a 29:71 mixture of diastereoisomers.

$^{1}$H nmr $\tau$($CDCl_3$):

8.95 (3H,d), 6.85-7.60 (10H, complex, 2H exchangeable), 5.40 (1H, q), 2.25-3.0 (8H, complex).

0089154

EXAMPLE 21

N-[2-(4-(2-Methoxyethylthio)phenyl)-1-methylethyl]-2-hydroxy-2-(3-chlorophenyl)ethanamine hydrochloride

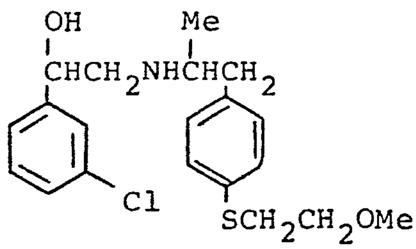

HCl

1-[4-(2-Methoxyethylthio)phenyl]propan-2-one (3.5g) and 2-hydroxy-2-(3-chlorophenyl)ethanamine (2.7g) were reacted together according to the procedure described in Example 2. Column chromatography gave an oil which was converted to its hydrochloride salt and crystallised from ethyl-acetate/diethyl ether to give N-[2-(4-(2-methoxyethyl-thio)phenyl)-1-methylethyl]-2-hydroxy-2-(3-chlorophenyl) ethanamine hydrochloride, m.p. 136-141$^{o}$C as a 34:66 mixture of diastereoisomers.

$^1H$ nmr τ ($(CD_3)_2SO$):

8.80 (3H,d), 6.35-7.45 (12H, complex), 4.80 (1H,q), 3.5 (1H, broad s, exchangeable), 2.3-2.85 (8H, complex), 0.2-1.2 (2H, broad s, exchangeable).

EXAMPLE 22

N-[2-(4-(2-Phenoxyethylthio)phenyl)-1-methylethyl]-2-hydroxy-2-(3-chlorophenyl) ethanamine

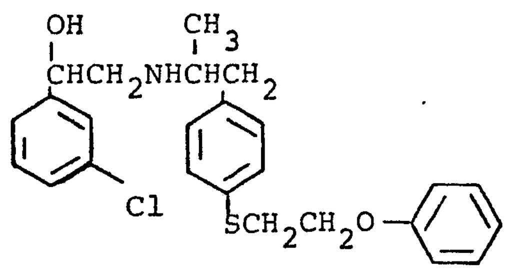

1-[4-(2-Phenoxyethylthio)phenyl]propan-2-one (4.8g) and 2-hydroxy-2-(3-chlorophenyl)ethanamine (2.9g) were reacted together according to the procedure described in Example 2. Column chromatography on silica gel gave an oil which was crystallised from diethyl ether to give N-[2-(4-(2-phenoxyethylthio)phenyl)1-methyethyl]-2-hydroxy-2-(3-chlorophenyl)ethanamine, m.p. 100-108$^{o}$C, as a 27:73 mixture of diastereoisomers.

$^{1}$H nmr $\tau$($CDCl_3$):

8.95 (3H,d), 6.20-8.00 (7H, complex, 2H exchangeable), 6.80 (2H,t), 5.90 (2H,t), 5.45 (1H,q), 2.5-3.3 (12H, complex).

EXAMPLE 23

N-[2-(4-(2-Methylaminoethylthio)phenyl)-1-methylethyl]-2-hydroxy-2-(3-chlorophenyl)ethanamine dihydrochloride

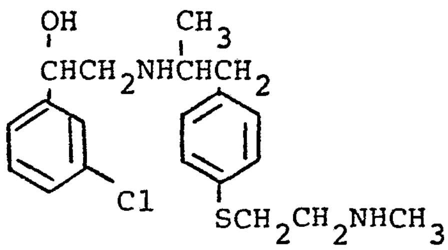

2HCl

1-[4-(2-Methylaminoethylthio)phenyl]propan-2-one (3.0g) and 2-hydroxy-2-(3-chlorophenyl)ethanamine (2.5g) were reacted together according to the procedure described in Example 2. Column chromatography on silica gel gave an oil which was converted to its dihydrochloride salt and crystallised from methanol/ethyl acetate to give N-[2-(4-(2-methylaminoethylthio)phenyl)-1-methylethyl]-2-hydroxy-2-(3-chlorophenyl) ethanamine dihydrochloride m.p. 188-192°C.

$^1H$ nmr $\tau((CD_3)_2SO)$:

8.90 (3H,d), 7.55 (3H,s), 6.40-7.45 (9H, complex), 4.90 (1H,q), 3.60 (1H,s, exchangeable), 2.40-2.80 (8H, complex), 0.40-0.85 (4H, broad s, exchangeable).

EXAMPLE 24

N-[2-(4-(2-Phenylthioethylthio)phenyl)-1-methylethyl]-2-hydroxy-2-(3-chlorophenyl)ethanamine

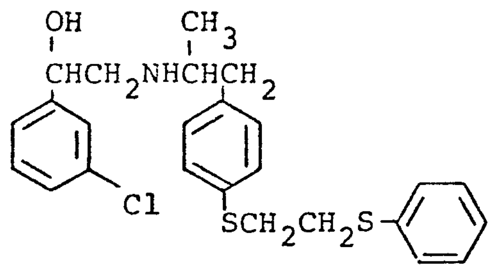

1-[4-(2-Phenylthioethylthio)phenyl]propan-2-one (5.2g) and 2-hydroxy-2-(3-chlorophenyl)ethanamine (3.0g) were reacted together according to the procedure described i Example 2. Column chromatography on silica gel and crystallisation from diethyl ether gave N-[2-(4-(2-phenylthi ethylthio)phenyl)-1-methylethyl]-2-hydroxy-2-(3-chlorophenyl)ethanamine, m.p. 97-104$^{o}$C, as a 32:68 mixture of diastereoisomers.

$^{1}$H nmr $\tau$($CDCl_3$):

8.95 (3H,d), 6.80-7.55 (11H, complex, 2H, exchangeable), 5.40 (1H,q), 2.50-3.15 (13H, complex).

Description 1

4-(2-Carboxyethylthio)benzaldehyde

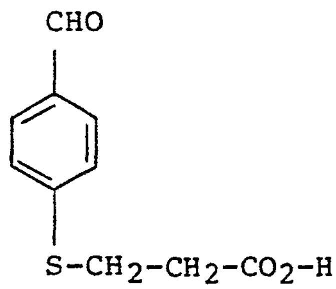

A mixture of 4-fluorobenzaldehyde (3.72 g), 3-mercaptopropionic acid (3.18 g) and potassium carbonate (7.0 g) in dimethylsulphoxide (80 ml) was stirred at 120°C for one hour and cooled. The reaction mixture was poured into water (300 ml) washed with dichloromethane, acidified and extracted with diethyl ether. The ethereal extracts were washed with water, dried (magnesium sulphate), filtered and the solvent removed under reduced pressure to give 4-(2-carboxyethylthio)- benzaldehyde, mp 101-3°C.

$^1H$ nmr; $\tau$ ($CDCl_3$) 7.35 (2H, t), 6.7 (2H, t), 2.65 (2H, d), 2.2 (2H, d), 0.1 (1H, s).

Description 2

4-(2-Carbomethoxyethylthio)benzaldehyde

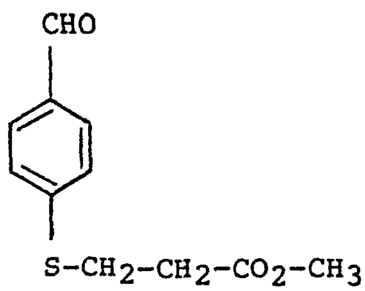

A mixture of 4-(2-carboxyethylthio)benzaldehyde (18.0 g), concentrated sulphuric acid (2 ml) and methanol (350 ml) was heated under reflux for 2 hours, cooled and the solvent removed under vacuum. The residue was poured into water (300 ml) and extracted with dichloromethane. The extracts were washed with aqueous sodium bicarbonate and dried (magnesium sulphate). The solvent was removed under reduced pressure to give 4-(2-carbomethoxythio)benzaldehyde as an oil which was used without further purification.

$^1$H nmr; τ ($CDCl_3$) 7.3 (2H, t), 6.7 (2H, t), 6.3 (3H, s), 2.6 (2H, d), 2.2 (2H, d), 0.05 (1H, s).

Description 3

1-[4-(2-Carbomethoxyethylthio)phenyl]-2-nitroprop-1-ene

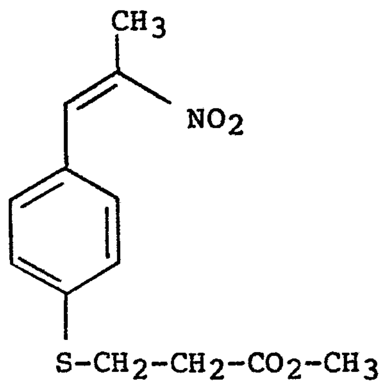

A mixture of 4-(2-carbomethoxyethylthio)-benzaldehyde (17.1 g) and n-butylamine (10 ml) in dry benzene (200 ml) was heated under reflux using a Dean and Stark head until the required amount of water had been collected. The mixture was cooled, and the solvent removed under reduced pressure. The residue was dissolved in acetic acid (60 ml); nitroethane (12 ml) added and the mixture stirred at 100-110°C for 2 hours, cooled and poured into water. Trituration of the mixture, followed by filtration and drying gave 1-[4-(2-carbomethoxyethylthio)phenyl]-2-nitroprop-1-ene, mp 49-51°C.

$^1H$ nmr; $\tau$ ($CDCl_3$) 7.5 (3H, s), 7.25 (2H, t), 6.7 (2H, t), 6.25 (3H, s), 2.5 (4H, s), 1.9 (1H, s).

Description 4

1-[4-(2-Carbomethoxyethylthio)phenyl]propan-2-one oxime

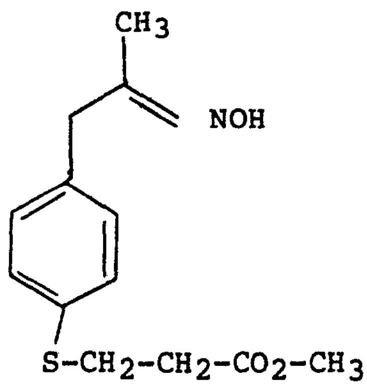

1-[4-(2-Carbomethoxyethylthio)phenyl]-2-nitroprop-1-ene (20 g) in tetrahydrofuran (250 ml) was stirred with aluminium amalgam (prepared from aluminium (18 g) and mercuric chloride (9 g)), with ice cooling until the reaction was complete. The slurry was filtered through diatomaceous earth and the filtrate evaporated to give 1-[4-(2-carbomethoxyethylthio)phenyl]propan-2-one oxime as an oil.

$^1$H nmr; τ (CDCl$_3$) 8.2 (3H, s), 7.4 (2H, m), 6.85 (2H, m), 6.3 (5H, two overlapping s), 2.5-3.0 (5H complex).

Description 5

1-[4-(2-Carbomethoxyethylthio)phenyl]propan-2-one

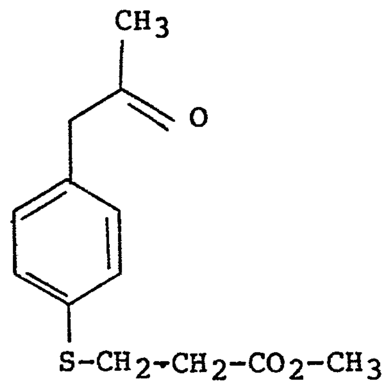

A solution of sodium metabisulphite (50 g) in water (120 ml) was added to 1-[4-(2-carbomethoxyethylthio)phenyl]propan-2-one oxime (17.4 g) in methanol (100 ml) and the mixture heated under reflux for 16 hours. The reaction mixture was cooled to 0°C, concentrated hydrochloric acid (50 ml) added and the mixture was extracted with ethyl acetate. The extracts were washed with aqueous sodium bicarbonate (5 times), water, dried (magnesium sulphate) and evaporated to dryness to give 1-[4-(2-carbomethoxyethylthio)-phenyl]propan-2-one as an oil.

$^1H$ nmr; $\tau$ ($CDCl_3$) 7.8 (3H, s), 7.35 (2H, t), 6.8 (2H, t), 6.3 (5H, s), 2.5-3.0 (4H, complex dd).

Description 6

4-Methylthiobenzaldehyde

To a solution of methylpotassium thiolate (8.6 g) in dimethylsulphoxide (80 ml) was added 4-fluorobenzaldehyde (12.4 g) and the mixture was stirred at 110°C for 2 hours. The reaction mixture was cooled and iced water (200 ml) was added. The aqueous mixture was extracted with ether, the ethereal extract was washed with saturated brine, dried (magnesium sulphate) and the solvent evaporated. Distillation of the residue gave 4-methylthiobenzaldehyde (7.65 g), bp 124-6°C (5 mm).

$^1H$ nmr; $\tau$ ($CDCl_3$) 6.15 (3H, s), 3.1 (2H, d, J=8Hz), 2.2 (2H, d, J=8Hz), 0.15 (1H, s).

Description 7

4-Isopropylthiobenzaldehyde

To a solution of 2-propanethiol (12.26 g) in dimethylsulphoxide (75 ml) containing potassium carbonate (22 g),4-fluorobenzaldehyde (20 g) was added and the mixture was heated at 100°C for 3 hours with stirring. After cooling the reaction mixture was poured into water, and the mixture extracted with ether. The extracts were dried (magnesium sulphate) and evaporated to dryness. Distillation gave 4-isopropylthiobenzaldehyde (24.5 g) bp 98-100°C (0.6 mm).

$^1H$ nmr; $\tau$ ($CDCl_3$) 8.65 (6H, d, J=6Hz), 6.4 (1H, m), 2.7 (2H, d, J=8Hz), 2.25 (2H, d, J=8Hz), 0.1 (1H, s).

Description 8

4-Benzylthiobenzaldehyde

4-Benzylthiobenzaldehyde was prepared according to the procedure in Description 7, from 4-fluorobenzaldehyde (9.92 g) and benzyl mercaptan (10.93 g), as a white solid, mp 63-4°C (diethyl ether/60-80° petroleum ether).

$^1H$ nmr; $\tau$ ($CDCl_3$) 5.8 (2H, s), 2.7 (5H, s), 2.65 (2H, d, J=8Hz), 2.3 (2H, d, J=8Hz), 0.15 (1H, s).

Description 9

1-(4-Methylthiophenyl)-2-nitroprop-1-ene

1-(4-Methylthiophenyl)-2-nitroprop-1-ene was prepared, according to the procedure described in Description 3, from 4-methylthiobenzaldehyde (5.0 g) as a yellow solid (5.6 g), mp 38-40°C.

$^1H$ nmr; $\tau$ ($CDCl_3$) 7.6 (3H, s), 7.5 (3H, s), 2.7 (4H, s), 2.0 (1H, s).

Description 10

1-(4-Isopropylthiophenyl)-2-nitroprop-1-ene

1-(4-Isopropylthiophenyl)-2-nitroprop-1-ene was prepared, according to the method described in Description 3, from 4-isopropylthiobenzaldehyde (24.4 g) as a red oil (31.1 g).

$^1H$ nmr; $\tau$ ($CDCl_3$) 8.65 (6H, d, J=6Hz), 7.55 (3H, s), 6.45 (1H, m), 2.5 (4H, s), 1.95 (1H, s).

Description 11

1-(4-Benzylthiophenyl)-2-nitroprop-1-ene

1-(4-Benzylthiophenyl)-2-nitroprop-1-ene was prepared, according to the method described in Description 3, from 4-benzylthiobenzaldehyde (13.5 g) as a yellow solid, mp 92-5°C.

$^1H$ nmr; $\tau$ ($CDCl_3$) 7.6 (3H, s), 5.85 (2H, s), 2.7 (9H, s), 2.05 (1H, s).

Description 12

1-(4-Methylthiophenyl)propan-2-one

To a mixture of 1-(4-methylthiophenyl)-2-nitroprop-1-ene (10 g) and iron powder (10 g) in tetrahydrofuran (100 ml) heated under reflux, concentrated hydrochloric acid (30 ml) was added, at such a rate that reflux was maintained. The reaction mixture was refluxed for a further 1 hour, cooled, filtered, water added and most of the organic solvent was removed by evaporation. The residue was extracted with ether, the extracts were dried (magnesium sulphate) and the solvent evaporated to yield 1-(4-methylthiophenyl)propan-2-one (7.1 g) as a colourless oil, which was used without further purification.

$^1H$ nmr; $\tau$ ($CDCl_3$) 7.8 (3H, s), 7.5 (3H, s), 6.3 (2H, s), 2.8 (2H, d, J=8Hz), 2.2 (2H, d, J=8Hz).

Description 13

1-(4-Isopropylthiophenyl)propan-2-one

1-(4-Isopropylthiophenyl)propan-2-one was prepared, according to the procedure described in Description 12, from 1-(4-isopropylthiophenyl)-2-nitroprop-1-ene (31 g) as a colourless oil (19 g), bp 112-20°C, (0.5 mm).

$^1$H nmr; τ ($CDCl_3$) 8.7 (6H, d, J=6Hz), 7.85 (3H, s), 6.6 (1H, m), 6.3 (2H, s), 2.9 (2H, d, J=8Hz), 2.6 (2H, d, J=8Hz).

Description 14

1-(4-Benzylthiophenyl)propan-2-one

1-(4-Benzylthiophenyl)propan-2-one was prepared, according to the procedure described in Description 12, from 1-(4-benzylthiophenyl)-2-nitroprop-1-ene (13.6 g), as a yellow oil (11.6 g) which was used without further purification.

$^1$H nmr; τ ($CDCl_3$) 7.9 (3H, s), 6.4 (2H, s), 5.9 (2H, s), 2.6-3.2 (9H, m).

Description 15

4-t-Butylthiobenzaldehyde

4-t-Butylthiobenzaldehyde, bp 96-8°C/0.6 mm, was prepared from t-butylmercaptan (14.52 g) and 4-fluorobenzaldehyde (20 g) by an analogous procedure to that described in Description 7.

$^1$H nmr; τ ($CDCl_3$) 8.65 (9H, s), 2.1-2.45 (4H, dd), -0.05 (1H, s).

Description 16

4-n-Heptylthiobenzaldehyde

4-n-Heptylthiobenzaldehyde, bp 145-8°C/0.4 mm, was prepared from n-heptylmercaptan (21.3 g) and 4-fluorobenzaldehyde (20 g) by an analogous procedure to that described in Description 7.

$^1$H nmr; τ ($CDCl_3$) 9.05-9.25 (3H, t), 8.15-8.95 (10H, complex), 7.05 (2H, t), 2.65 (2H, d), 2.25 (2H, d), 0.05 (1H, s).

Description 17

1-(4-t-Butylthiophenyl)-2-nitroprop-1-ene

1-(4-t-Butylthiophenyl)-2-nitroprop-1-ene was obtained as a red oil from 4-t-butylthiobenzaldehyde by an analogous procedure to that described in Description 3.

$^1$H nmr; τ ($CDCl_3$) 8.65 (9H, s), 7.55 (3H, s), 2.2-2.8 (4H, dd), 1.95 (1H, s).

Description 18

1-(4-n-Heptylthiophenyl)-2-nitroprop-1-ene

1-(4-n-Heptylthiophenyl)-2-nitroprop-1-ene was prepared, as an oil, from 4-n-heptylthiobenzaldehyde by an analogous procedure to that described in Description 3.

$^1$H nmr; τ ($CDCl_3$) 8.1-9.3 (13H, complex), 7.55 (3H, s), 7.0 (2H, t), 2.4-2.9 (4H, complex), 1.95 (1H, s).

## Description 19

### 1-(4-t-Butylthiophenyl)propan-2-one

1-(4-t-Butylthiophenyl)propan-2-one, bp 136-8°C/0.5 mm, was prepared from 1-(4-t-butylthiophenyl)-2-nitroprop-1-ene by an analogous procedure to that described in Description 12.

$^1H$ nmr; τ ($CDCl_3$) 8.7 (9H, s), 7.85 (3H, s), 6.3 (2H, s), 2.2-2.9 (4H, dd).

## Description 20

### 1-(4-n-Heptylthiophenyl)propan-2-one

1-(4-n-Heptylthiophenyl)propan-2-one, bp 178-80°C/0.5 mm, was prepared from 1-(4-n-heptylthiophenyl)-2-nitroprop-1-ene by an analogous procedure to that described in Description 12.

$^1H$ nmr; τ ($CDCl_3$) 8.1-9.2 (13H, complex), 7.8 (3H, s), 7.1 (2H, t), 6.3 (2H, s), 2.5-3.0 (4H, dd).

## Description 21

### 1-(4-Methylsulphonylphenyl)-2-nitroprop-1-ene

1-(4-Methylsulphonylphenyl)-2-nitroprop-1-ene was prepared from 4-methylsulphonylbenzaldehyde by an analogous procedure to that described in Description 3.

$^1H$ nmr; τ ($CDCl_3$) 7.5 (3H, s), 6.7 (3H, s), 2.2 (2H, d), 1.9 (2H, d), 1.8 (1H, s).

Description 22

1-(4-Methylsulphonylphenyl)propan-2-one

1-(4-Methylsulphonylphenyl)propan-2-one was prepared from 1-(4-methylsulphonylphenyl)-2-nitroprop-1-ene by an analogous procedure to that described in Description 12.

$^1$H nmr; $\tau$($CDCl_3$) 7.75 (3H, s), 6.90 (3H, s), 6.15 (2H, s), 2.65 (2H, d), 2.05 (2H, d).

Description 23

1-(4-Isopropylthiophenyl)propan-2-one ethylene ketal

A mixture of 1-(4-isopropylthiophenyl)propan-2-one (9.5 g), excess ethanediol, and p-toluenesulphonic acid (catalytic amount) in benzene was heated under reflux using a Dean and Stark head for 2 hours, cooled, washed with water, dried (magnesium sulphate), filtered and evaporated to dryness. Distillation of the residue gave 1-(4-isopropylthiophenyl)propan-2-one ethylene ketal as a yellow liquid (7.6 g), bp 124-8°C/0.8 mm.

$^1$H nmr; $\tau$($CDCl_3$) 8.65 (6H, d), 8.6 (3H, s), 7.1 (2H, s), 6.5 (1H, m), 6.0-6.3 (4H, m), 2.7 (4H, m).

## Description 24

### 1-(4-Isopropylsulphonylphenyl)propan-2-one

Hydrogen peroxide (100 vol; 8 ml) was added to 1-(4-isopropylthiophenyl)propan-2-one ethylene ketal (3.6 g) in glacial acetic acid (15 ml) and stirred at 0-5°C for one hour then at ambient temperature overnight. The solution was poured into water and extracted with ethyl acetate. The extracts were washed with aqueous sodium bicarbonate solution, dried and evaporated to dryness. The residue was dissolved in acetone, dilute hydrochloric acid added and the mixture stirred for 24 hours. After removal of acetone under reduced pressure, and neutralisation with aqueous sodium bicarbonate, extraction with ethyl acetate and evaporation of the extracts gave 1-(4-isopropylsulphonylphenyl)propan-2-one as a colourless oil which was used without further purification.

## Description 25

### 1-[4-(2-Carbomethoxyethylsulphonyl)phenyl]propan-2-one

1-[4-(2-Carbomethoxyethylsulphonyl)phenyl]propan-2-one was prepared from 1-[4-(2-carbomethoxyethylthio)-phenyl]propan-2-one by an analogous sequence to that described in Descriptions 23 and 24.

$^1H$ nmr; $\tau$ ($CDCl_3$) 7.70 (3H, s), 7.25 (2H, t), 6.7 (2H, t), 6.45 (3H, s), 6.3 (2H, s), 2.6 (2H, d), 2.2 (2H, d).

Description 26

1-(4-Isopropylsulphonylmethylphenyl)propan-2-one

Sulphur dioxide gas was passed through a solution of isopropylmagnesium iodide (prepared from isopropyl iodide (12.58 g) and magnesium (1.79 g)) in dry diethyl ether (100 ml) for 20 minutes. Ice and water were added to the resultant mixture. After decanting the ethereal phase, the aqueous phase was added to a mixture of 1-(4-bromomethylphenyl)propan-2-one ethylene ketal (10 g) in methanol (75 ml), heated under reflux for six hours and allowed to stand overnight. The solvent was removed in a vacuum and the residue extracted with ethyl acetate, dried and evaporated to dryness. Column chromatography on silica gel (ether-hexane as eluent) gave 1-(4-isopropylsulphonylmethylphenyl)propan-2-one as a white solid.

$^1H$ nmr; $\tau$ ($CDCl_3$) 8.60 (6H, d), 7.9 (3H, s), 7.0 (1H, m), 6.30 (2H, s), 5.8 (2H, s), 2.5-3.0 (4H, m).

Description 27

1-(4-Methylsulphonylmethylphenyl)propan-2-one

1-(4-Methylsulphonylmethylphenyl)propan-2-one was prepared by an analogous procedure to that described in Description 26, except that methyl iodide was used in place of isopropyl iodide.

0089154

1-(4-Isopropylthiomethylphenyl)propan-2-one

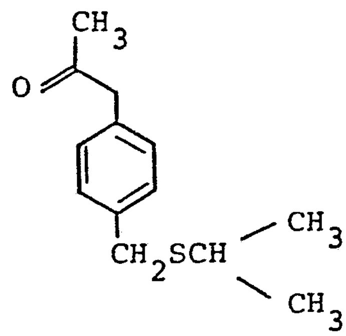

To a solution of 1-(4-hydroxymethylphenyl)propan-2-one ethylene ketal (2.08g) in dichloromethane (20ml), was added 2-propane thiol (0.9ml), boron trifluoride-etherate (2ml), and the resulting mixture stirred at room temperature for 3 days. Evaporation of the solvent *in vacuo* gave an oil which was chromatographed on silica using dichloromethane as eluent to give 1-(4-isopropylthiophenyl)propan-2-one.

$^1$H nmr $\tau$($CDCl_3$):

8.75 (6H,d), 7.86 (3H,s), 7.2 (1H,m), 6.35 (2H,s), 6.3 (2H,s), 2.5-2.95 (4H,m).

DESCRIPTION 29



1-(4-n-Heptylsulphonylphenyl)propan-2-one

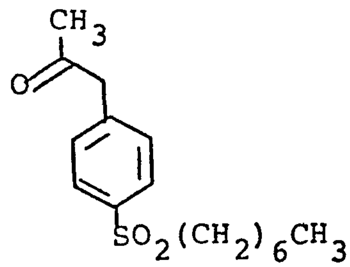

1-(4-n-Heptylsulphonylphenyl)propan-2-one was prepared from 1-(4-n-heptylthiophenyl)propan-2-one by an analogous sequence to that described in Descriptions 23 and 24.

$^1$H nmr $\tau$($CDCl_3$):

9.1 (3H,t), 8.0-9.0 (10H,m), 7.7 (3H,s), 6.9 (2H,t), 6.1 (2H,s), 2.6 (2H,d), 2.15 (2H,d).

DESCRIPTION 30

4-(Methylsulphinylphenyl)propan-2-one

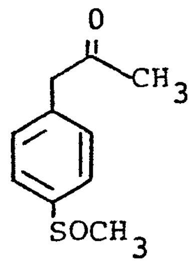

To a solution of 4-(methylthiophenyl)propan-2-one (3.0g) in dichloromethane (50ml) was added metachloroperbenzoic acid (3.1g), portionwise with stirring and ice cooling. The mixture was stirred for 30 minutes at $0^{o}C$, 30 minutes at room temperature, then washed with saturated sodium bicarbonate solution, dried (magnesium sulphate) and evaporated to give 4-(methylsulphinylphenyl)propan-2-one as an oil.

$^{1}H$ nmr $\tau$ ($CDCl_3$):

7.85 (3H,s), 7.30 (3H,s), 6.20 (2H,s), 2.65 (2H,d), 2.40 (2H,d).

DESCRIPTION 31



4-(2-Hydroxyethylthio)benzaldehyde

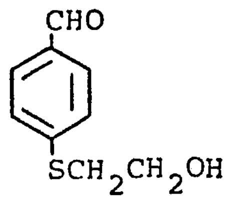

4-Fluorobenzaldehyde (12.4g) and 2-mercaptoethanol (7.8g) were reacted together as described in Description 7 to give 4-(2-hydroxyethylthio)benzaldehyde, m.p. 59-62°C.

$^1H$ nmr $\tau(CDCl_3)$:

7.1 (1H, broad s, exchangeable), 6.75 (2H,t), 6.15 (2H,t), 2.65 (2H,d), 2.30 (2H,d), 0.2 (1H,s).

DESCRIPTION 32



1-[4-(2-Hydroxyethylthio)phenyl]-2-nitroprop-1-ene

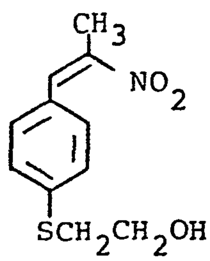

1-[4-(2-Hydroxyethylthio)phenyl]-2-nitroprop-1-ene was prepared according to the procedure described in Description 3 from 4-(2-hydroxyethylthio)-benzaldehyde.

$^1H$ nmr $\tau(CDCl_3)$

7.60 (3H,s), 7.20 (1H,s, exchangeable), 6.85 (2H,t), 6.25 (2H,t), 2.70 (4H,s), 2.00 (1H,s).

DESCRIPTION 33

1-[4-(2-Hydroxyethylthio)phenyl]propan-2-one ethylene ketal

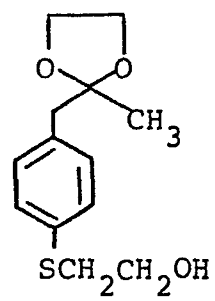

1-[4-(2-Hydroxyethylthio)phenyl]propan-2-one was prepared from 1-[4-(2-hydroxyethylthio)phenyl]-2-nitroprop-1-ene according to the method described in description 3, and 1-[4-(2-hydroxyethylthio)phenyl)propan-2-one ethylene ketal was obtained by an analogous procedure to that described in Description 23.

$^1$H nmr $\tau$ ($CDCl_3$):

8.72 (3H,s), 7.20 (2H,s), 6.9-7.2 (3H, complex, 1H exchangeable), 6.0-6.55 (6H, complex), 2.60-3.00 (4H, complex).

DESCRIPTION 34

1-[4-(2-Tosyloxyethylthio)phenyl]propan-2-one ethylene ketal

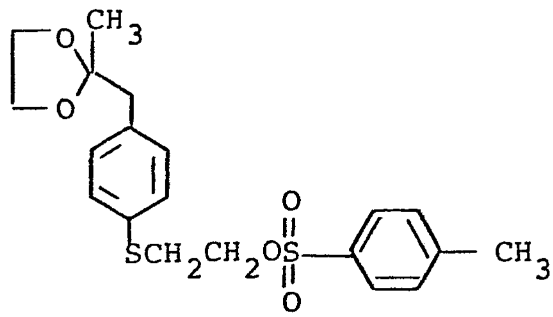

To an ice cold solution of 1-[4-(2-hydroxyethylthio)phenyl] propan-2-one ethylene ketal (27g) in pyridine (70ml) was added 4-toluenesulphonyl chloride (28g) portionwise with stirring. On completion of the addition the mixture was stirred at room temperature for 1 hour. The mixture was added to water, acidified with 10% hydrochloric acid and extracted twice with dichloromethane. The organic extracts were washed with 10% hydrochloric acid, dried (magnesium sulphate) and evaporated to give an oil which solidified on standing.

$^1H$ nmr $\tau$ $CDCl_3$:

8.72 (3H,s), 7.57 (3H,s), 3.17 (2H,s), 5.4-6.9 (8H,complex), 2.0-3.6 (8H, complex).

1-[4-(2-Methoxyethylthio)phenyl]propan-2-one

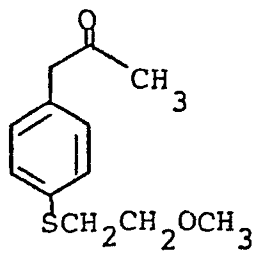

A solution of 1-[4-(2-tosyloxyethylthio)phenyl]-propan-2-one ethylene ketal (8.16g) in methanol (50ml) was added to a solution of sodium (0.5g) in methanol (30ml). The mixture was heated under reflux for 5 hours, cooled and evaporated to dryness. Water (100ml) was added, extracted with ethyl acetate, dried (magnesium sulphate), filtered and evaporated under reduced pressure to yield an oil. This oil in acetone (50ml) was treated with 10% hydrochloric acid (30ml) and allowed to stand at room temperature for 1 hour. The solvent was removed under reduced pressure, water (100ml) added and the mixture was extracted with ethyl acetate three times. The combined organic extracts were dried (magnesium sulphate), filtered and evaporated to give 1-[4-(2-methoxyethylthio)phenyl]propan-2-one as an oil which was used without further purification.

$^1$H nmr $\tau$($CDCl_3$):

7.87 (3H,s), 7.00 (2H,t), 6.57 (2H,s), 6.3-6.7 (5H, complex) 2.95 (2H,d), 2.70 (2H,d).

DESCRIPTION 36



1-[4-(2-Phenoxyethylthio)phenyl]propan-2-one

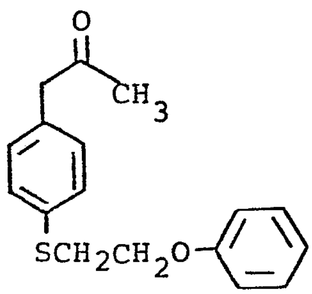

A solution of 1-[4-(2-tosyloxyethylthio)phenyl]propan-2-one ethylene ketal (8.16g) in ethanol (50ml) was added to a solution of sodium phenoxide (2.32g) in ethanol (50ml) and the mixture was stirred and heated under reflux for 4 hours. After cooling, the solvent was evaporated, the residue was suspended in water (100ml) and the mixture was extracted twice with ether. The combined organic extracts were dried (magnesium sulphate), filtered and evaporated to give an oil which was deketalised as in Description 35 to give 1-[4-(2-phenoxyethylthio)phenyl]propan-2-one, m.p. 35-38$^{o}$C.

$^{1}$H nmr $\tau$ ($CDCl_3$):

7.85 (3H,s), 6.95 (2H,t), 6.35 (2H,s), 5.90 (2H,t), 2.5-3.3 (9H, complex).

1-[4-(2-Methylaminoethylthio)phenyl]propan-2-one

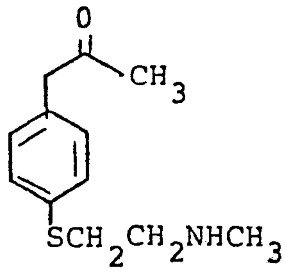

Methylamine was passed through a solution of 1-[4-(2-tosyloxyethylthio)phenyl]propan-2-one (8.5g) in dimethyl sulphoxide for 3 hours. The solution was stirred at room temperature overnight. The mixture was poured into iced water (250ml), extracted with ether, the organic extracts washed with saturated sodium chloride solution, dried (magnesium sulphate) and evaporated to dryness. The product in acetone (100ml) was treated with 5% hydrochloric acid (50ml) and allowed to stand at room temperature for 1 hour. Water (100ml) was added and the mixture was extracted with ether. The aqueous phase was basified with saturated sodium bicarbonate solution, extracted twice with ether and the combined organic extracts dried (magnesium sulphate), filtered and evaporated to give 1-4-(2-methylaminoethylthio)phenyl]propan-2-one as an oil.

$^1H$ nmr $\tau(CDCl_3)$:

7.85 (3H,s), 7.6 (1H,s,exchangeable), 6.8-7.3 (5H,complex), 6.2-6.5 (4H,complex), 2.95 (2H,d), 2.70 (2H,d).

DESCRIPTION 38

0089154

1-[4-(2-Phenylthioethylthio)phenyl]propan-2-one

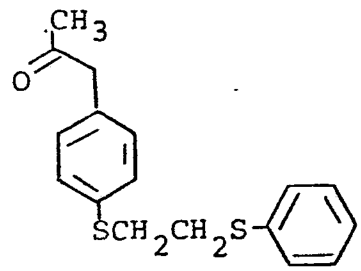

1-[4-(2-Phenylthioethylthio)phenyl]propan-2-one, m.p. 60-62°C, was prepared from 1-[4-(2-tosyloxyethylthio)phenyl) propan-2-one and sodium thiophenolate in a similar process to that described in Description 36.

$^1H$ nmr $\tau$ $(CDCl_3)$:

7.85 (3H,s), 6.90 (4H,s), 6.35 (2H,s), 2.30-3.10 (9H, complex).

## Demonstration of Effectiveness of Compounds

### (i) Effect on energy expenditure

The effect of the compounds on the energy expenditure of mice was demonstrated by means of the following procedure:

Female CFLP mice each weighing approximately 24 g, were given food and water ad lib before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content, following the principles described by J.B. de V. Weir, J. Physiol. (London) 109, 1-9 (1949). The results are expressed as a rate of energy expenditure of the mice dosed with water.

| COMPOUND OF EXAMPLE NO | DOSE (mg/kg po) | MEAN ENERGY EXPENDITURE % (0-3h) | (0-21h) |
|---|---|---|---|
| 1 | 24.6 | 116 | 102 |
| 2 | 18.7 | 150 | 112 |
| 3 | 22.3 | 110 | 105 |
| 4 | 23.0 | 107 | 98 |
| 5 | 25.5 | 178 | 122* |
| 6 | 27.9 | 159 | 119 |
| 7 | 22.3 | 146 | 101+ |
| 8 | 23.9 | 134 | 108 |
| 9 | 24.8 | 123 | 105 |
| 10 | 24.5 | 144 | 113 |
| 11 | 23.1 | 134 | 108 |
| 12 | 24.7 | 146 | 109 |
| 13 | 21.1 | 169 | 121 |
| 14 | 22.9 | 168 | 122 |
| 15 | 25.5 | 116 | 108 |
| 16 | 25.2 | 124 | 104 |
| 17 | 18.3 | 143 | 110 |
| 18 | 18.6 | 142 | 114 |
| 19 | 10.4 | 146 | 107 |
| 20 | 22.8 | 155 | 117 |
| 21 | 23.2 | 138 | 115 |
| 22 | 22.1 | 153 | 112 |
| 23 | 25.1 | 146 | 109 |
| 24 | 22.8 | 128 | 110 |

* 0 to 19 hours
\+ 0 to 18 hours.

(ii) Cardiac activity

Rat hearts were perfused by the Langendorff procedure. Hearts were dissected free within 30 seconds of death and reverse perfused via the aorta and coronary vessels with Krebs-Ringer bicarbonate solution (pH 7.4, 37°C) gassed with 95% oxygen:5% carbon dioxide at a flow rate between 8-12 $cm^3$/minute. Responses were observed after injection of drug dissolved in isotonic saline into the perfusion media. Heart rate and tension were displayed on an Ormed MX2P recorder via a tension transducer and heart ratemeter.

Results are expressed as a percentage of the maximum response due to salbutamol.

| COMPOUND OF EXAMPLE NO. | DOSE ADDED TO PERFUSATE (μg) | HEART TENSION | HEART RATE |
|---|---|---|---|
| 1 | 30 | 4 | 0 |
| 2 | 30 | 25 | 23 |
| 7 | 30 | 0 | 25 |
| 8 | 30 | 30 | 29 |
| 10 | 30 | 91 | 31 |
| 11 | 30 | 18 | 67 |
| 12 | 30 | 40 | 13 |

(iii) Anti-hyperglycaemic activity

Female CFLP mice, weighing approximately 25 g, were fasted for 24 hours prior to the study. The compounds under study were administered orally as an aqueous solution to each of 6 mice. 30 Minutes later a blood sample (10 µl) was obtained from the tail for the analysis of blood glucose. Immediately after taking this blood sample, glucose (1 g/kg body weight) was administered subcutaneously to each mouse. 6 Mice were given water as a control. Blood samples were then obtained from each mouse at 30 minute intervals for 120 minutes.

Compounds that produced a significant (P 0.05) reduction of blood glucose, compared with control mice given water, at any time interval, were considered active. The area under the blood glucose curve over the 2 hour period after the administration of the glucose was calculated for each compound and compared with the value for control animals.

| COMPOUND OF EXAMPLE NO. | DOSE (µmol/kg) | % REDUCTION IN AREA UNDER BLOOD GLUCOSE CURVE |
|---|---|---|
| 1 | 12.5 | 35 |
| 2 | 1.0 | 8 |
| 3 | 0.5 | 28 |
| 4 | 12.5 | 17 |
| 7 | 12.5 | 48 |
| 9 | 12.5 | 52 |
| 10 | 1.0 | 10 |
| 15 | 100.0 | 48 |
| 17 | 2.5 | 30 |
| 18 | 5.0 | 16 |
| 19 | 2.5 | 26 |
| 20 | 12.5 | 59 |
| 21 | 1.0 | 31 |
| 22 | 2.5 | 23 |
| 23 | 1.0 | 16 |
| 24 | 2.5 | 12 |

(iv) Anti-inflammatory Activity

The method used is based on that described by G.Tonelli et al (Endocrinology, 77, 625-634, 1965). An inflammation is induced in the rat ear by the application of 50 µl of a 1% solution of croton oil in tetrahydrofuran, test compounds being dissolved in the irritant vehicle. After 6 hours the inflammation is assessed by killing the animals and weighing the ears. Topical anti-inflammatory activity of a compound is generally considered to be shown when a significant (5% level) reduction in ear weight is seen compared to non-drug treated control.

| Compound of Example No. | Dose mg/rat ear | Activity |
|---|---|---|
| 1 | 1.00 | Active (89% inhibition) |

Toxicity

No toxicity was observed during the above experiments.

CLAIMS

0089154

1. A compound of formula (I), or a salt thereof:

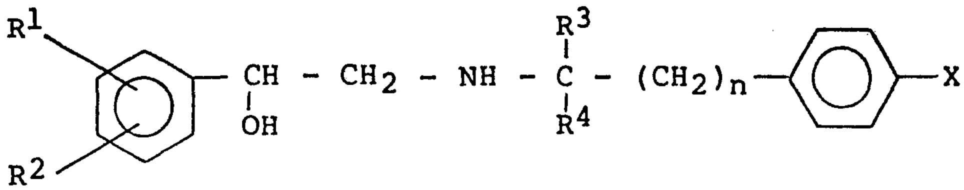

(I)

wherein;

$R^1$ is hydrogen, halogen or trifluoromethyl,

$R^2$ is hydrogen or halogen,

$R^3$ is hydrogen or methyl,

$R^4$ is hydrogen or methyl,

n is 1 or 2

X is $-Y-SR^5$, 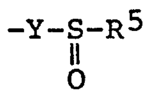 or 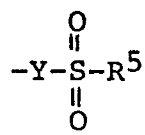

$R^5$ is optionally substituted benzyl or $C_{1-12}$ straight or branched alkyl optionally substituted by carboxy, $C_{1-6}$ alkoxycarbonyl, benzyloxycarbonyl, aminocarbonyl, mono- or di-$(C_{1-6})$alkylamino-carbonyl, hydroxy, $C_{1-6}$ alkoxy, benzyloxy, phenoxy, amino, mono- or di-$(C_{1-6})$alkylamino, $C_{1-6}$ alkylthio, benzylthio or phenylthio

provided that, when $R^5$ is alkyl substituted by hydroxy, alkoxy, alkylthio, phenylthio, benzylthio, amino, alkylamino or benzyloxy, such substituents are separated from the sulphur atom by at least two carbon atoms, and

Y is methylene or a bond.

2. A compound according to claim 1, in which $R^1$ is m-Cl or m-$CF_3$ and $R^2$ is hydrogen.

3. A compound according to claim 1 or claim 2, in which one of $R^3$ and $R^4$ is hydrogen and the other is methyl.

4. A compound according to claim 1, selected from

N-[2-(4-(2-Carbomethoxyethylthio)phenyl)-1-methylethyl] 2-hydroxy-2-(3-trifluoromethylphenyl)-ethanamine;
N-[2-(4-Methylthiophenyl)-1-methylethyl]-2-hydroxy-2-(3-chlorophenyl)ethanamine;
N-[2-(4-Isopropylthiophenyl)-1-methylethyl]-2-hydroxy-2-(3-chlorophenyl)ethanamine hydrochloride;
N-[2-(4-Benzylthiophenyl)-1-methylethyl]-2-hydroxy-2-(3-chlorophenyl)ethanamine;
N-[2-(4-t-Butylthiophenyl)-1-methylethyl]-2-hydroxy-2-(3-chlorophenyl)ethanamine hydrobromide;
N-[2-(4-n-Heptylthiophenyl)-1-methylethyl]-2-hydroxy-2-(3-chlorophenyl)ethanamine hydrobromide;
N-[2-(4-Methylsulphonylphenyl)-1-methylethyl]-2-hydroxy-2-(3-trifluoromethylphenyl)ethanamine;
N-[2-(4-Isopropylsulphonylphenyl)-1-methylethyl] -2-hydroxy-2-(3-trifluoromethylphenyl)ethanamine;
N-[2-(4- 3-Hydroxypropylsulphonyl phenyl)-1-methylethyl]-2-hydroxy-2-(3-trifluoromethylphenyl)ethanamine;
N-[2-(4- 2-Carbomethoxyethylsulphonyl phenyl)-1-methylethyl]-2-hydroxy-2-(3-chlorophenyl)-ethanamine;
N-[2-(4-Methylsulphonylmethylphenyl)-1-methylethyl]-2-hydroxy-2-(3-trifluoromethylphenyl)-ethanamine;
N-[2-(4-Isopropylsulphonylmethylphenyl)-1-methylethyl]-2-hydroxy-2-(3-trifluoromethylphenyl)-ethanamine;

N-[2-(4-isopropylthiomethylphenyl)-1-methylethyl]-2-hydroxy-2-(3-chlorophenyl)ethanamine;

N-[2-(4-isopropylthiomethylphenyl)-1-methylethyl]-2-hydroxy-2-(3-chlorophenyl)ethanamine hydrobromide;

N-[2-(4-t-butylsulphonylphenyl)-1-methylethyl]- 2-hydroxy-2-(3-chlorophenyl)ethanamine hydrobromide;

N-[2-(4-n-heptylsulphonylphenyl)-1-methylethyl]-2-hydroxy-2-(3-chlorophenyl)ethanamine;

N-[2-(4-isopropylthiophenyl)-1-methylethyl]-2-hydroxy-2-phenylethanamine;

(RS:SR)-N-[2-(4-methylthiophenyl)-1-methylethyl]-2-hydroxy-2-(3-chlorophenyl)ethanamine;

(RR,SS)-N-[2-(4-methylthiophenyl)-1-methylethyl]-2-hydroxy-1-(3-chlorophenyl)ethanamine hydrochloride;

N-[2-(4-methylsulphinylphenyl)-1-methylethyl]-2-hydroxy-2-(3-chlorophenyl)ethanamine;

N-[2-(4-(2-methoxyethylthio)phenyl)-1-methylethyl]-2-hydroxy-2-(3-chlorophenyl) ethanamine hydrochloride;

N-[2-(4-(2-phenoxyethylthio)phenyl)-1-methylethyl]-2-hydroxy-2-(3-chlorophenyl)ethanamine;

N-[2-(4-(2-methylaminoethylthio)phenyl)-1-methylethyl]-2-hydroxy-2-(3-chlorophenyl)ethanamine dihydrochloride;

N-[2-(4-(2-phenylthioethylthio)phenyl)-1-methylethyl]-2-hydroxy-2-(3-chlorophenyl)ethanamine.

5. A process for producing a compound of formula (I) as defined in claim 1, or a salt thereof, which comprises (a) reducing an oxo-group and/or double bond and/or cleaving an N-benzyl group of a compound of formula (II):

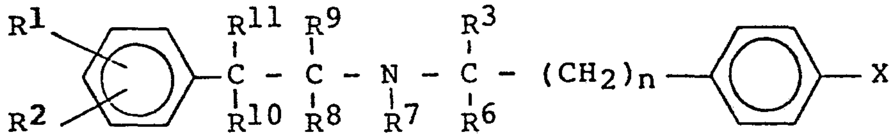

(II)

wherein $R^1$, $R^2$, $R^3$, X and n are as defined in relation to formula (I),

$R^6$ is a group $R^4$ as defined in relation to formula (I) or together with $R^7$ forms a bond,

$R^7$ is hydrogen or benzyl or together with $R^6$ or $R^8$ forms a bond,

$R^8$ is hydrogen or together with $R^9$ forms an oxo-group or together with $R^7$ forms a bond,

$R^9$ is hydrogen or together with $R^8$ forms an oxo-group,

$R^{10}$ is hydroxyl or together with $R^{11}$ forms an oxo-group,

$R^{11}$ is hydrogen or together with $R^{10}$ forms an oxo-group

provided that there is no more than one oxo-group represented by any of $R^8$ to $R^{11}$; and optionally thereafter forming a salt of the compound of formula (I) so formed and/or converting the compound of formula (I) so formed into a further compound of formula (I);

or (b) reacting a compound of formula (III):

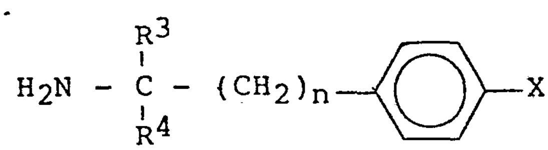

(III)

with a compound of formula (IV):

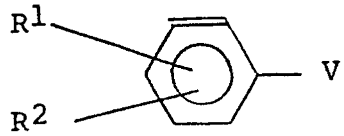

(IV)

wherein $R^1$, $R^2$, $R^3$, $R^4$, n and X are as defined in formula (I); and V is a group capable of reacting with the compound of formula (III) to form a compound of formula (I), and optionally thereafter forming a salt of the compound of formula (I) so formed, or

(c) reacting a compound of formula (V):

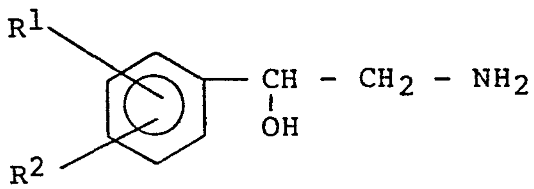

(V)

with a compound of formula (VI):

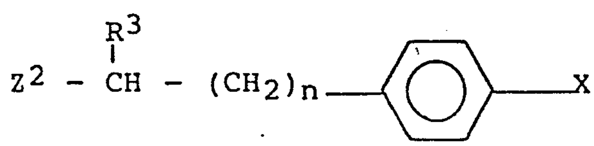

(VI)

wherein $R^1$, $R^2$, $R^3$, n and X are as defined in formula (I) and $Z^2$ is a leaving group, and optionally thereafter forming a salt of the compound of formula (I), so formed.

6. A pharmaceutical composition comprising a compound according to any one of claims 1 to 4 and a pharmaceutically acceptable carrier therefor.

7. A composition according to claim 6 in unit dosage form.

8. A compound according to any one of claims 1 to 4 for use in treating the human or animal body.